# EUROPEAN PATENT SPECIFICATION

(11) **EP 4 555 958 B1**
(45) Date of publication and mention of the grant of the patent: **28.10.2026**
(21) Application number: 24168327.5
(22) Date of filing: 03.04.2024
(51) Int. Cl.: A61B 18/20

(54) **SKIN TREATMENT APPARATUS**
HAUTBEHANDLUNGSVORRICHTUNG
APPAREIL DE TRAITEMENT DE LA PEAU

(30) Priority: 17.11.2023 CN 202323141550 U
(43) Date of publication of application: 21.05.2025
(73) Proprietor: Shenzhen Ulike Smart Electronics Co., Ltd., Shenzhen, Guangdong 518000 (CN)
(72) Inventor: HE, Dengshi, Shenzhen (CN)
(74) Representative: Winter, Brandl - Partnerschaft mbB

(56) References cited:
- CN-A- 112 168 344
- CN-A- 112 206 051
- CN-A- 117 442 331
- JP-A- 2023 052 801
- US-A1- 2022 192 745
- US-A1- 2022 346 871

## Description

### TECHNICAL FIELD

The present disclosure relates to the technical field of skin treatment, and in particular, to a skin treatment apparatus.

### BACKGROUND

Hair removal devices and skin rejuvenation devices are skin treatment devices that are commonly used in people's lives. The skin treatment devices mainly make use of intense pulsed light (IPL) or laser light to irradiate users' skin, to achieve the effect of hair removal or rejuvenation for users.

In the case of a hair removal device, the hair removal device includes a housing, an IPL light source, and a cold compress component. The housing is provided with a light outlet area. The IPL light source is arranged in the housing and is configured to generate light irradiated to skin from the light outlet area. The cold compress component is disposed at the light outlet area and is configured to cool the skin.

In related art, heat dissipation of the light source and the cold compress component is required to improve the safety of use. However, the heat dissipation effect of the skin treatment device in related art needs to be improved.

US 2022192745 A1 discloses a portable depilation instrument including a shell, and a depilation mechanism and a heat dissipation mechanism inside the shell. The shell is provided with at least two outlets and one inlet, the shell introduces an external cooling medium from the inlet, the heat dissipation mechanism includes a heat conductive component, the depilation mechanism includes a refrigeration element and an emitter, the heat conductive component isolates the refrigeration element from the emitter in two mutually sealed channels, namely, a first channel and a second channel, the first channel and the second channel are respectively in communication with two outlets, and one of the outlets allows the first channel or the second channel to pass through inside of the entire shell from one end to the other end. Due to complete isolation and a push force, air can be discharged only from a respective corresponding outlet after a heat exchange.

### SUMMARY

The present disclosure is set out in the appended set of claims.

Embodiments of the present disclosure provide a skin treatment apparatus, which can improve the heat dissipation effect of the skin treatment apparatus.

The embodiments of the present disclosure provide a skin treatment apparatus, including:
a housing provided with a light outlet area;
a light-emitting component disposed in the housing, the light-emitting component being configured to generate light irradiated to a skin to be treated from the light outlet area;
a cold compress component disposed at the light outlet area for cooling the skin; and
a heat dissipation component, including a fan, a temperature equalizing plate, and a heat dissipation sheet mounted on the temperature equalizing plate. The fan is defined with a first air vent, one end of the temperature equalizing plate is thermally connected to the cold compress component, the other end of the temperature equalizing plate is located at the first air vent, the fan drives a first part of air to flow through a side of the temperature equalizing plate facing away from the heat dissipation sheet and flow through the light-emitting component to dissipate heat from the light-emitting component, and drives a second part of air to flow through the heat dissipation sheet to dissipate heat from the cold compress component.

**In** some embodiments, the skin treatment apparatus further includes a holder component disposed in the housing. The holder component and the temperature equalizing plate define a first heat dissipation channel for the first part of air to flow through. The light-emitting component is at least partially disposed in the first heat dissipation channel. The holder component includes a separation part, and the separation part separates the light-emitting component from the temperature equalizing plate.

**In** some embodiments, the separation part is shorter than the temperature equalizing plate along a direction of the light-emitting component facing the first air vent, and the separation part is supported on the temperature equalizing plate; and/or, a side surface of the temperature equalizing plate facing the light-emitting component includes a sheltered area and an exposed area, the separation part is supported on the sheltered area, and a minimum distance between the exposed area and the light-emitting component is greater than or equal to 6 millimeters; and/or, a side surface of the temperature equalizing plate facing the light-emitting component includes a sheltered area and an exposed area, the separation part is supported on the sheltered area, and the exposed area is a flat area for guiding and dissipating heat; and/or, the skin treatment apparatus is a hair removal device or a skin rejuvenation device.

In some embodiments, the skin treatment apparatus further includes a holder component disposed in the housing, and the holder component and the temperature equalizing plate define a first heat dissipation channel and a second heat dissipation channel. The light-emitting component is at least partially mounted in the first heat dissipation channel, one side of the temperature equalizing plate and the holder component define a near air vent section of the first heat dissipation channel, the near air vent section of the first heat dissipation channel is communicated with the first air vent, and the fan drives the first part of air to flow through the near air vent section of the first heat dissipation channel to dissipate heat from the light-emitting component. The heat dissipation sheet is disposed in the second heat dissipation channel, the other side of the temperature equalizing plate and the holder component define a near air vent section of the second heat dissipation channel, the near air vent section of the second heat dissipation channel is communicated with the first air vent, and the fan drives the second part of air to flow through the near air vent section of the second heat dissipation channel to dissipate heat from the cold compress component.

In some embodiments, the light outlet component extends along a length direction of the first air vent. The near air vent section of the first heat dissipation channel and the near air vent section of the second heat dissipation channel are communicated to different areas of the first air vent along a width direction.

In some embodiments, the first heat dissipation channel includes a mounting section and a flow guiding section arranged along a direction of the light-emitting component facing the first air vent. The light-emitting component is at least partially mounted in the mounting section. One end of the flow guiding section is located at the first air vent to be communicated with the first air vent, and the other end of the flow guiding section is communicated with the mounting section. The near air vent section of the first heat dissipation channel is defined in the flow guiding section. The cross-sectional area of at least partial region of the flow guiding section gradually increases along a direction of the first air vent facing the light-emitting component.

In some embodiments, an inner wall of the flow guiding section includes a first inner side wall facing the temperature equalizing plate, and a second inner side wall facing the first inner side wall. Along the direction of the first air vent facing the light-emitting component, at least one of the first inner side wall and the second inner side wall is inclined away from the other one, to allow a distance between the first inner side wall and the second inner side wall to be gradually increased.

In some embodiments, a width of an end of the flow guiding section close to the light-emitting component is greater than or equal to a width of the light-emitting component in a direction of the first inner wall facing the second inner wall.

In some embodiments, a first anti-turbulence structure is provided in the flow guiding section to limit a formation of turbulence in the flow guiding section.

In some embodiments, the light-emitting component includes a light tube and a light reflective member. The light reflective member includes an arc-shaped part disposed around the light tube, and the arc-shaped part is at least partially located on a side of the light tube close to the flow guiding section. One end of the arc-shaped part in a peripheral direction is adjacent to the first inner side wall, and the other end of the arc-shaped part in the peripheral direction is adjacent to the second inner side wall. The first inner side wall or the second inner side wall is provided with the first anti-turbulence structure, or each of the first inner side wall and the second inner side wall is provided with the first anti-turbulence structure.

In some embodiments, the first anti-turbulence structure includes a flow guiding plate. The flow guiding plate extends along the direction of the first air vent facing the light-emitting component. And/or, the flow guiding plate is one of a plurality of flow guiding plates, and the plurality of flow guiding plates are spaced apart along a length direction of the light-emitting component. And/or, the flow guiding plate includes a first inclined surface and a second inclined surface facing each other along a thickness direction; the first inclined surface and the second inclined surface are inclined away from each other along the direction of the first air vent facing the light-emitting component, to allow a thickness of an end of the flow guiding plate close to the light-emitting component to be greater than a thickness of an end of the flow guiding plate away from the light-emitting component. And/or, an inner wall of the flow guiding section includes a first flow guiding surface connected with the flow guiding plate, the flow guiding plate further includes a third inclined surface, the third inclined surface is located on a side of the flow guiding plate facing away from the first flow guiding surface; the third inclined surface is inclined away from the first flow guiding surface along the direction of the first air vent facing the light-emitting component, to allow a height of the flow guiding plate protruding from the first flow guiding surface at an end of the flow guiding plate close to the light-emitting component to be greater than a height of the flow guiding plate protruding from the first flow guiding surface at an end of the flow guiding plate away from the light-emitting component.

In some embodiments, an end of the temperature equalizing plate close to the first air vent is deflected toward a side close to the light-emitting component, to form a deflection section. The deflection section extends to the first air vent, a side of the deflection section close to the light-emitting component and the holder component define at least part of the flow guiding section, and a side of the deflection section facing away from the light-emitting component and the holder component define the near air vent section of the second heat dissipation channel.

In some embodiments, the heat dissipation sheet includes a proximal heat dissipation section mounted on the deflection section, and a distal heat dissipation section connected to an end of the proximal heat dissipation section close to the light outlet area. A width of the proximal heat dissipation section is greater than a width of the distal heat dissipation section, and a width of the heat dissipation sheet is a distance from a side of the heat dissipation sheet close to the temperature equalizing plate to a side of the heat dissipation sheet away from the temperature equalizing plate.

In some embodiments, the heat dissipation sheet includes a connection side edge mounted on the temperature equalizing plate, and a free side edge facing away from the temperature equalizing plate. Along the direction of the light-emitting component facing the first air vent, the connection side edge and the free side edge of the proximal heat dissipation section are inclined away from each other, to allow a width of the proximal heat dissipation section to be gradually increased.

In some embodiments, the housing includes a light outlet section extending from a junction of the proximal heat dissipation section and the distal heat dissipation section to the light outlet area. A cross-sectional area of the light outlet section gradually decreases along a direction of the light-emitting component facing the light outlet area.

In some embodiments, there are a plurality of heat dissipation sheets, each heat dissipation sheet includes the connection side edge mounted on the temperature equalizing plate and the free side edge facing away from the temperature equalizing plate, the free side edges of the plurality of heat dissipation sheets define a free side surface, and the free side surface includes an air blocking area close to the first air vent and a circulation area close to the cold compress component. The heat dissipation component further includes a blocking part, the blocking part blocks the air blocking area, and air between adjacent heat dissipation sheets flows out or flows into the circulation area close to the cold compress component.

In some embodiments, a length of the blocking part accounts for more than one-third of a length of each heat dissipation sheet along the direction of the first air vent facing the light-emitting component.

In some embodiments, the blocking part at least blocks the free side edges of the proximal heat dissipation section.

In some embodiments, the blocking part includes an air baffle, and the air baffle covers the free side edges of the plurality of heat dissipation sheets; and/or, the blocking part includes a plurality of folded edges, and each folded edge is bendably connected to the free side edge of one heat dissipation sheet.

In some embodiments, the housing is further defined with a second air vent communicated with an outside of the housing, the second air vent is located on a side of the heat dissipation sheet facing away from the temperature equalizing plate, the second air vent is disposed corresponding to the blocking part, and the second air vent is communicated with the circulation area.

In some embodiments, a second anti-turbulence structure is disposed on the side of the heat dissipation sheet away from the temperature equalizing plate. The second anti-turbulence structure is located in the circulation area, to prevent air flowing out of the circulation area from forming turbulence.

In some embodiments, each heat dissipation sheet is provided with the second anti-turbulence structure. And/or, the second anti-turbulence structure includes a plurality of tooth parts, and the plurality of tooth parts are protruded on the side of the heat dissipation sheet facing away from the temperature equalizing plate. And/or, the second anti-turbulence structure includes a plurality of tooth parts, which are triangular.

In some embodiments, the housing is further defined with a second air vent communicated with an outside of the housing, and the second air vent is located on a side of the heat dissipation sheet facing away from the temperature equalizing plate; an end of the first heat dissipation channel away from the first air vent is communicated with the second air vent so as to be communicated with the outside of the housing; and an end of the second heat dissipation channel facing away from the first air vent is communicated with the second air vent so as to be communicated with the outside of the housing.

In some embodiments, the housing is further defined with a second air vent communicated with an outside of the housing, and the second air vent is located on a side of the heat dissipation sheet facing away from the temperature equalizing plate. An inner wall of the housing further defines a third heat dissipation channel, one end of the third heat dissipation channel is communicated with an end of the first heat dissipation channel away from the first air vent, and the other end of the third heat dissipation channel and an end of the second heat dissipation channel away from the first air vent are both communicated with the second air vent and form a Bernoulli structure at the junction.

In some embodiments, an outlet at an end of the third heat dissipation channel close to the second air vent is defined in an inner wall at an end of the second heat dissipation channel close to the second air vent.

In some embodiments, the second heat dissipation channel extends from the first air vent of the fan to the second air vent of the housing, the heat dissipation sheet is disposed in the second heat dissipation channel, and the heat dissipation sheet is adjacent to both the first air vent and the second air vent. The first heat dissipation channel is located on a side of the temperature equalizing plate facing away from the second air vent, to allow a total length of a flow path of air in the first heat dissipation channel and the third heat dissipation channel to be greater than a total length of a flow path of air in the second heat dissipation channel.

In some embodiments, the skin treatment apparatus further includes a circuit board component. The circuit board component includes a circuit board disposed in the housing, the circuit board and the holder component define the third heat dissipation channel; and a conductive holder mounted on the circuit board and located in the third heat dissipation channel. The light-emitting component includes a light tube, an end of the light tube being connected to the conductive holder, to allow the light tube to be supported on the circuit board and electrically connected with the circuit board.

In some embodiments, the skin treatment apparatus further includes a circuit board component. The circuit board component includes a circuit board and a conductive holder. The circuit board is disposed in the housing, and conductive holder is mounted on the circuit board. The light-emitting component includes: a light tube, and an end of the light tube is connected with the conductive holder, to allow the light tube to be supported on the circuit board and electrically connected with the circuit board; and a light reflective member disposed around the light tube, the light reflective member being electrically connected with the circuit board, and a distance between the light reflective member and the light tube being less than a preset distance, to allow the light reflective component to excite the light tube.

In some embodiments, the circuit board component further includes a separation member sleeved on at least one of the light tube and the conductive holder, and the separation member separates the light reflective member from the conductive holder. And/or, there are at least two light tubes, and the at least two light tubes are arranged at intervals.

In some embodiments, an inner wall of the first heat dissipation channel is defined with a mounting port, the light-emitting component is mounted in the first heat dissipation channel through the mounting port, and the light-emitting component and an inner wall of the mounting port define a gap air vent. The light-emitting component includes a light reflective member and a light source, the light source is disposed in the light reflective member, a side air vent is defined in a peripheral side of the light reflective member, the side air vent is communicated with the first heat dissipation channel, and an end of the light reflective member is further defined with an end air vent.

In some embodiments, the end air vent is adjacent to the gap air vent to form a Bernoulli structure.

In some embodiments, the light source includes a light tube. The light reflective member is disposed on the peripheral side of the light tube. Along the length direction of the light tube, an end of the light reflective member is disposed through the mounting port, and an outer wall of the light reflective member and an inner wall of the mounting port define the gap air vent. Along the length direction of the light tube, the end air vent is defined between the inner wall of the light reflective component and the light tube, to allow the gap air vent and the end air vent to form a Bernoulli structure.

In some embodiments, the light outlet area and the first air vent are located on different sides in the peripheral direction of the light tube; and/or an opening facing the light outlet area is defined in two sides of the light reflective member, to gather light generated by the light tube to the light outlet area, where the side air vent is defined on at least one side of the light reflective member; and/or the mounting port is defined in an inner wall of the first heat dissipating air channel in at least one end of the length direction of the light tube, and the mounting ports on different sides of the light tube in the length direction are communicated to the second heat dissipating air channel through different third heat dissipating air channels.

In some embodiments, the light-emitting component includes a light tube mounted in the mounting section. An inner wall of the flow guiding section further includes a third inner side wall and a fourth inner side wall, the third inner side wall and the fourth inner side wall are both connected between the first inner side wall and the second inner side wall, and the third inner side wall and the fourth inner side wall are located at different ends in a length direction of the light tube. Along the direction of the first air vent facing the light-emitting component, at least one of the third inner side wall and the fourth inner side wall is inclined close to the other one to guide part of air flowing out of the first air vent to the light-emitting component.

In some embodiments, in a length direction of the light tube, a length of an end of the flow guiding section close to the light-emitting component is less than a length of the light tube.

In some embodiments, the holder component is further defined with at least one third heat dissipation channel. The third heat dissipation channel is disposed on a side of the third inner wall facing away from the fourth inner wall and/or on a side of the fourth inner wall facing away from the third inner wall. The third heat dissipation channel extends from the first inner wall toward the second inner wall. One end of the third heat dissipation channel is communicated with the end of the first heat dissipation channel away from the first air vent, and the other end of the third heat dissipation channel is communicated with the second heat dissipation channel.

In the embodiments of the present disclosure, since the fan drives the first part of air and the second part of air to flow through different sides of the temperature equalizing plate, the contact area between the temperature equalizing plate and the cooling airflow is increased. This improves the heat dissipation efficiency of the temperature equalizing plate, which in turn improves the heat dissipation effect of the cold compress component, thereby ultimately enhancing the overall heat dissipation effect of the skin treatment apparatus.

### BRIEF DESCRIPTION OF DRAWINGS

The specific embodiments of the present disclosure are described in detail with reference to the attached drawings, to make the technical solution of the present disclosure and its beneficial effects obvious.
FIG. 1 is a schematic structural diagram of a skin treatment apparatus according to an embodiment of the present disclosure.
FIG. 2 is a sectional diagram of the skin treatment apparatus of FIG. 1 along A-A direction.
FIG. 3 is a schematic diagram of mounting positions of a light-emitting component, a cold compress component, and a heat dissipation component of the skin treatment apparatus in FIG. 2.
FIG. 4 is an enlarged diagram of part X in FIG. 2.
FIG. 5 is an enlarged diagram of part X in FIG. 2 showing another flow diverting structure.
FIG. 6 is another sectional diagram of the skin treatment apparatus of FIG. 1 along A-A direction.
FIG. 7 is an enlarged diagram of part Y in FIG. 6.
FIG. 8 is another enlarged diagram of the part X in FIG. 2.
FIG. 9 is a schematic structural diagram of a first blocking part at the heat dissipation sheets of the skin treatment apparatus in FIG. 2.
FIG. 10 is a schematic structural diagram of a first blocking part at the heat dissipation sheets of the skin treatment apparatus in FIG. 2.
FIG. 11 is a sectional diagram of the skin treatment apparatus in FIG. 2 along B-B direction.
FIG. 12 is a sectional diagram of the heat dissipation component and the light-emitting component of FIG. 2.
FIG. 13 is another sectional diagram of the skin treatment apparatus of FIG. 1.
FIG. 14 is a sectional diagram of the skin treatment apparatus of FIG. 2 along C-C direction.
FIG. 15 is an enlarged diagram of part Z in FIG. 14.
FIG. 16 is a schematic diagram of a first circuit board component of the skin treatment apparatus in FIG. 2.
FIG. 17 is a schematic diagram of a second circuit board component of the skin treatment apparatus in FIG. 2.
FIG. 18 is a schematic diagram of a third circuit board component of the skin treatment apparatus in FIG. 2.
FIG. 19 is a schematic structural diagram of an insulated gate bipolar transistor (IGBT) member of the skin treatment apparatus in FIG. 2.
FIG. 20 is a schematic structural diagram of FIG. 2 showing another fan of the skin treatment apparatus.
FIG. 21 is a schematic structural diagram of the skin treatment apparatus in FIG. 1 from another perspective.
FIG. 22 is a sectional diagram of the skin treatment apparatus of FIG. 21 along D-D direction.

### DESCRIPTION OF EMBODIMENTS

The following clearly and completely describes the technical solution in the embodiments of the present disclosure with reference to the accompanying drawings. Obviously, the described embodiments are only a part of the embodiments of the present disclosure, rather than all of them. Based on the embodiments of the present disclosure, all other embodiments obtained by those skilled in the art without making creative efforts shall fall within the protection scope of the present disclosure.

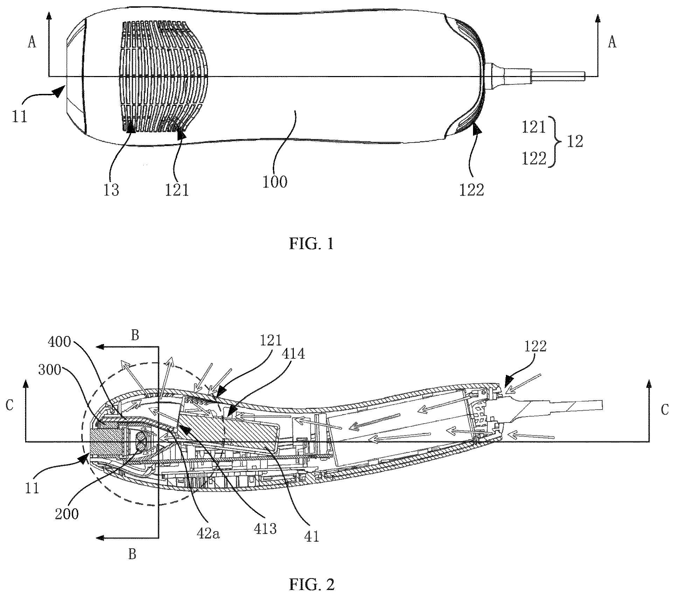

Referring to FIG. 1 and FIG.2, FIG. 1 is a schematic structural diagram of a skin treatment apparatus according to an embodiment of the present disclosure, and FIG. 2 is a sectional diagram of the skin treatment apparatus of FIG. 1 along A-A direction. The embodiments of the present disclosure provide a skin treatment apparatus.

Skin treatment apparatus is an apparatus that adjusts and improves the skin condition of the body and face based on the physiological function of the human body. It has functions of whitening, skin rejuvenation, freckle removal, wrinkle removal, hair removal, and the like. Based on optics, various effects can be achieved by irradiating the skin with special wavelengths of light or different types of light. For example, intense pulsed light (IPL), light-emitting diode (LED) light, and laser are widely used today.

For example, the skin treatment apparatus is a hair removal device or a skin rejuvenation device.

The skin treatment apparatus may include a housing 100, a light-emitting component 200, a cold compress component 300, and a heat dissipation component 400.

The housing 100 is provided with a light outlet area 11. The light-emitting component 200 is disposed in the housing 100, and is configured to generate light irradiated to a skin to be treated from the light outlet area 11. The cold compress component 300 is disposed at the light outlet area 11 and is configured to cool the skin. The heat dissipation component 400 is configured to dissipate heat from the light-emitting component 200 and/or the cold compress component 300.

Depending on the type of light produced by the light-emitting component 200, the skin treatment apparatus may be categorized into different types. For example, in case the light-emitting component 200 is configured to generate the laser light, namely the light-emitting component 200 is a laser light source component, then the skin treatment apparatus is a laser skin treatment apparatus. For another example, in case the light-emitting component 200 is configured to generate the IPL light, namely the light-emitting component 200 is an IPL light source component (such as a xenon light component), then the skin treatment apparatus is an IPL skin treatment apparatus; for still another example, the light-emitting component 200 is an LED light-emitting component.

In the present disclosure, the light-emitting component 200 being an IPL light source component is described below as an example. The light-emitting principle of this type of skin treatment apparatus is: a capacitor is connected with a power supply, and is charged by boosting a transformer component. When the capacitor is charged to a preset value and a controller receives a trigger signal, the electric energy in the capacitor is released, such that an instantaneous voltage can reach several hundred volts, which excites a light tube to instantly release intense pulse light, completing one light emission.

In some embodiments, the light outlet area 11 may be an opening or a hole defined in the housing 100.

In some other embodiments, the light outlet area 11 may be defined by some light-transmitting areas of the housing 100. For example, at least part of the housing 100 of the skin treatment apparatus, such as the head part, may be made of a light-transmitting material, a local area of the head may be provided with a light-shielding layer, and the other area that is not provided with the light-shielding layer defines the light outlet area 11. The present disclosure is not limited thereto.

The heat dissipation component 400 includes a fan 41. The fan 41 is defined with a first air vent 413. The first air vent 413 is disposed on a side of the light-emitting component 200 facing away from the light outlet area 11.

A flow diverting structure 42a may be provided in the housing 100. The flow diverting structure 42a is disposed at the first air vent 413, to allow the fan 41 to drive a first part of air to flow through one side of the flow diverting structure 42a to dissipate heat from the light-emitting component 200, and drive a second part of air to flow through the other side of the flow diverting structure 42a to dissipate heat from the heat dissipation component 300.

For example, in some embodiments, the first air vent 413 may be an air outlet, such that the flow diverting structure 42a can divide the air blown out from the fan 41 into the first part of air and the second part of air, to dissipate the heat from the light-emitting component 200 and the heat dissipation component 300. This allows heat of the light-emitting component 200 and the heat dissipation component 300 to be effectively dissipated, thereby improving the overall heat dissipation effect of the skin treatment apparatus. Certainly, the first air vent 413 may be an air inlet of the fan 41, which is not limited in the present disclosure.

The fan 41 may be a centrifugal fan, a cross-flow fan, or the like, which is not limited in the present disclosure.

For example, in case the fan 41 is a centrifugal fan, the first air vent 413 may be an air outlet or an air inlet of a volute, or may be an air outlet of an air channel component mounted at the air inlet or at the air outlet of the volute, which is not limited in the present disclosure.

In case the fan 41 is a centrifugal fan, the width direction of the first air vent 413 may be the thickness direction of the volute.

In some embodiments, the fan 41 is further defined with a third air outlet 414. One of the first air vent 413 and the third air vent 414 is the air inlet of the fan 41, and the other one of the first air vent 413 and the third air vent 414 is the air outlet of the fan 41. Correspondingly, the housing 100 is further defined with a fourth air vent 12, which is communicated with the outside of the housing 100 and the third air vent 414.

In case the first air vent 413 is the air outlet, the fan 41 first sucks the air outside the housing 100 through the third air outlet 414 and the fourth air vent 12; then, the sucked air flows out of the fan 41 through the first air vent 413 and is divided by the flow diverting structure 42a, so as to realize the heat dissipation of the heat dissipation component 300 and the light-emitting component 200.

For example, the fourth air vent 12 may include a first housing air inlet 121, which is disposed corresponding to (e.g., directly faces) the air inlet of the fan 41.

The fourth air vent 12 may further include a second housing air inlet 122, which is disposed at an end of the housing 100 facing away from the light outlet area 11.

The above is an overall description of the technical solution of the embodiments of the present disclosure. In the following, the technical solution of the embodiments of the present disclosure is described with reference to an optional structure of the flow diverting structure 42a.

It can be understood that the flow diverting structure 42a may be a part of the heat dissipation component 400, or may be a part of another component of the skin treatment apparatus, which is not limited in the present disclosure.

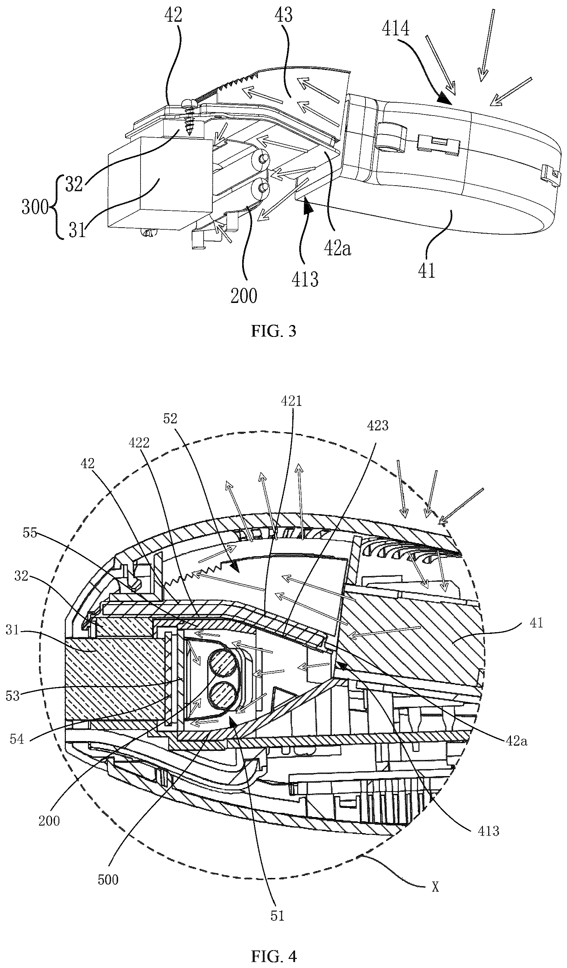

Illustratively, referring to FIG. 3 and FIG. 4, FIG. 3 is a schematic diagram of mounting positions of a light-emitting component, a cold compress component, and a heat dissipation component of the skin treatment apparatus in FIG. 2, and FIG. 4 is an enlarged diagram of part X in FIG. 2. The heat dissipation component 400 may further include a temperature equalizing plate 42 and a heat dissipation sheet 43 mounted on the temperature equalizing plate 42. One end of the temperature equalizing plate 42 is thermally connected to the cold compress component 300, for example, one end of the temperature equalizing plate 42 is directly attached to the cold compress component 300 or is attached to the cold compress component 300 by a thermal conductive medium such as thermal conductive silicone grease, such that the heat at the cold compress component 300 can be uniformly transferred to various positions of the temperature equalizing plate 42. Moreover, the contact area with the air is increased by the heat dissipation sheet 43, which improves the heat dissipation efficiency of the cold compress component 300. The other end of the temperature equalizing plate 42 is located at the first air vent 413 to form at least part of the flow diverting structure 42a. This can also be understood as that the other end of the temperature equalizing plate 42 being located at the first air vent 413 allows the fan 41 to drive the first part of air to flow through the side of the temperature equalizing plate 42 facing away from the heat dissipation sheet 43 and flow through the light-emitting component 200 to dissipate the heat from the light-emitting component 200, and drive the second part of air to flow through the heat dissipation sheet 43 to dissipate the heat of the cold compress component 300.

Since the fan 41 drives the first part of air and the second part of air to flow through different sides of the temperature equalizing plate 42, the contact area between the temperature equalizing plate 42 and the cooling airflow is increased. This improves the heat dissipation efficiency of the temperature equalizing plate 42, which in turns improves the heat dissipation effect of the cold compress component 300, thereby ultimately enhancing the overall heat dissipation effect of the skin treatment apparatus.

It can also be understood that the temperature equalizing plate 42 forming at least part of the flow diverting structure 42a means that the flow diverting structure 42a may be formed only by the temperature equalizing plate 42, or may be formed by the temperature equalizing plate 42 and other parts, which is not limited in the present disclosure.

The structure of the cold compress component 300 is described below for the purpose of explaining the principle of the temperature equalizing plate 42 dissipating the heat from the cold compress component 300.

In some embodiments, the cold compress component 300 being disposed at the light outlet area 11 may be that the cold compress component 300 passes through the light outlet area 11, or that the cold compress component 300 is disposed at an end face for the opening of the light outlet area 11.

For example, the cold compress component 300 may include a first light guiding member 31 and a refrigeration sheet 32. The first light guiding member 31 is disposed at the light outlet area 11 to transmit the light emitted by the light-emitting component 200. The refrigeration sheet 32 is thermally connected to the first light guiding member 31. For example, the refrigeration sheet 32 is directly attached to the first light guiding member 31 or is attached to the first light guiding member 31 by a thermal conductive medium such as heat conducting silicone grease.

In this way, the first light guiding member 31 can be cooled by the refrigeration sheet 32, and the cooled first light guiding member 31 is exposed from the light outlet area 11 to be in contact with the skin. Therefore, the first light guiding member 31 can transmit the light of the light source 22 for maintaining the user's skin, and the refrigeration sheet 32 can further apply cool compress treatment on the user's skin.

Specifically, the refrigeration sheet 32 is also called a semiconductor refrigeration sheet. Depending on the Peltier effect of semiconductor materials, when a direct current passes through a couple made of two different semiconductor materials in series, heat can be absorbed and released at two ends of the couple, thereby achieving refrigeration.

The first light guiding member 31 may be made of a sapphire material, or other light guiding material, which is not limited in the present disclosure.

Optionally, the cold compress component 300 may include a cold compress member (not shown in the figures) and a refrigeration sheet 32. The cold compress member is disposed on one side or the peripheral side of the light outlet area 11. The refrigeration sheet 32 is thermally connected to the cold compress member. For example, the refrigeration sheet 32 is directly attached to the cold compress member or is attached to the cold compress member by a thermal conductive medium such as thermal conductive silicone grease. Therefore, the cold compress member can be cooled by the refrigeration sheet 32, and the cooled cold compress member can be exposed from one side or the peripheral side of the light outlet area 11 to be in contact with the skin, so as to apply the cold compress treatment on the skin.

For example, the cold compress member may be an annular metal member disposed around the light outlet area 11.

The temperature equalizing plate 42 being thermally connected to the cold compress component 300 may be that the temperature equalizing plate 42 is thermally connected to the refrigeration sheet 32. For example, the temperature equalizing plate 42 is directly attached to the refrigeration sheet 32 or is attached to the refrigeration sheet 32 by a thermal conductive medium such as thermal conductive silicone grease. In this way, the heat at the refrigeration sheet 32 can be uniformly and rapidly transferred to various parts of the temperature equalizing plate 42. Moreover, the contact area between the temperature equalizing plate 42 and the air is increased by the heat dissipation sheet 43, thereby achieving rapid heat dissipation.

In the case of the refrigeration sheet 42 forming at least part of the flow diverting structure 42a, the skin treatment apparatus may further include a holder component 500 which is disposed inside the housing 100. The holder component 500 and the temperature equalizing plate 42 define a first heat dissipation channel 51 and a second heat dissipation channel 52. The light-emitting component 200 is at least partially mounted in the first heat dissipation channel 51, and the first part of air flows into the first heat dissipation channel 51 to dissipate the heat from the light-emitting component 200. The heat dissipation sheet 43 is disposed in the second heat dissipation channel 52, and the second part of air flows into the second heat dissipation channel 52 to dissipate the heat from the cold compress component 300. By dissipating the heat from the light-emitting component 200 and the cold compress component 300 separately, the heat dissipation effects of light-emitting component 200 and the cold compress component 300 are both ensured.

For example, one side of the temperature equalizing plate 42, such as the side facing away from the heat dissipation sheet 43, and the holder component 500 cooperatively define a near air vent section of the first heat dissipation channel 51. The near air vent section of the first heat dissipation channel 51 is communicated with the first air vent 413, so that the fan 41 is configured to/able to drive the first part of air to flow through the first heat dissipation channel 51 to dissipate the heat from the light-emitting component 200. The other side of the temperature equalizing plate 42, such as the side close to the heat dissipation sheet 43, and the holder component 500 cooperatively define a near air vent section of the second heat dissipation channel 52. The near air vent section of the second heat dissipation channel 52 is communicated with the first air vent 413, so that the fan 41 is able to drive the second part of air to flow through the second heat dissipation channel 52 to dissipate the heat from the cold compress component 300. Therefore, by defining the near air vent section of the first heat dissipation channel 51 and the near air vent section of the second heat dissipation channel 52 on two sides of the temperature equalizing plate, the first part of air can dissipate the heat from the light-emitting component 200, and at the same time, dissipate the heat from the temperature equalizing plate 42, which multiplexes the heat dissipation of the cold compress component 300.

In some embodiments, corresponding grooves and ribs may be provided in the holder component 500 for mounting the light-emitting component 200, the cold compress component 300, and at least part of the heat dissipation component 400.

In some embodiments, the inner wall of the first heat dissipation channel 51 may be provided with a light outlet part 53. The light outlet part 53 is located between the light-emitting component 200 and the light outlet area 11 of the housing 100. The light outlet part 53 is configured to allow light generated by the light-emitting component 200 to be transmitted to the light outlet area 11.

For example, the light outlet part 53 may be a through hole. The cold compress component 300, such as the first light guiding member 31 of the cold compress component 300, is disposed through the light outlet part 53, so as to seal the light outlet part 53.

Alternatively, the light outlet part 53 may be a second light guiding member, which is disposed on a side of the inner wall of the first heat dissipation channel 51 facing the light outlet area 11.

The second light guiding member may be a thermal-insulated light-transmitting member, which is configured to prevent heat generated by the light-emitting component 200 from being directly transferred to the cold compress component 300. Certainly, the second light guiding member may not be thermal-insulated, which is not limited in the present disclosure.

Alternatively, the second light guiding member is a filter.

The holder component 500 may further include a filter member 54. The filter member 54 may include a filter. The filter member 54 is configured to transmit light whose wavelength is ranged from 560 nanometers (nm) to 1200 nm, so as to form the IPL whose wavelength is within a specific range for irradiation on the user's skin. In actual use, the wavelength of the light required for hair removal may be ranged from 560 nm to 1200 nm, and the wavelength of the light required for skin rejuvenation may be ranged from 640 nm to 1200 nm. Therefore, the skin treatment apparatus has two functions of cold compress and skin rejuvenation, or cold compress and hair removal. Of cause, other functions may also be realized by arranging of the light-emitting component 200, or the light-emitting component 200 and the filter.

In some embodiments, the type of the filter may be changed, and the second light guiding member, the filter member 54, and the first light guiding member 31 are arranged in an order along the direction away from the light-emitting component 200, so as to provide the skin treatment apparatus with three functions of cold compress, skin rejuvenation, and hair removal.

In some embodiments, in the case of the skin treatment apparatus further including the holder component 500, the holder component 500 is disposed in the housing 100. The holder component 500 and the temperature equalizing plate 42 define the first heat dissipation channel 51 for the first part of air to flow through. The light-emitting component 200 is at least partially disposed in the first heat dissipation channel 51, and the first part of air flows through the light-emitting component 200 to dissipate heat from the light-emitting component 200. The holder component 500 may further include a separation part 55, which separates the light-emitting component 200 from the temperature equalizing plate 42.

It can be understood that, the temperature equalizing plate 42 may include a copper alloy temperature equalizing plate, a vapor chamber (VC) temperature equalizing plate, a stainless-steel temperature equalizing plate, a graphene heat dissipation plate, and the like, which is not limited in the present disclosure. Generally, the temperature equalizing plate 42 is electrically conductive. The separation part 55 allows the temperature equalizing plate 42 to be close to the light-emitting component 200, making the skin treatment apparatus as a whole lighter and thinner and preventing the distance between the light-emitting component 200 and the temperature equalizing plate 42 from being too close to cause an electricity leakage, thereby improving the reliability and safety of the skin treatment apparatus.

The separation part 55 may be made of an insulation material, such as plastic, rubber, ceramic, or an insulating layer, which is not limited in the present disclosure.

In some embodiments, along the direction of the light-emitting component 200 facing the first air vent 413, the separation part 55 is shorter than the temperature equalizing plate 42, and is supported on the temperature equalizing plate 42. Since the separation part 55 is relatively short, on one hand, the manufacturing difficulty and the manufacturing cost of the holder component 500, and the overall weight of the skin treatment apparatus can be reduced; on the other hand, the temperature equalizing plate 42 can be more exposed, to increase the contact area between the temperature equalizing plate 42 and the first part of air, which improves the heat dissipation efficiency of the temperature equalizing plate 42, ultimately improving the heat dissipation effect of the heat dissipation component 300.

In some embodiments, the side surface of the temperature equalizing plate 42 facing the light-emitting component 200 includes a sheltered area 422 and an exposed area 423. The separation part 55 is supported on the sheltered area 422. The minimum distance between the exposed area 423 and the light-emitting component 200 is greater than or equal to 6 millimeters (mm), preventing the distance between the temperature equalizing plate 42 and the light-emitting component 200 from being too close to cause the electricity leakage.

For example, the minimum distance between the exposed area 423 and the light-emitting component 200 may be 6 mm, 6.1 mm, 6.7 mm, 7.4 mm, 8 mm, or 9 mm, which is not limited in the present disclosure.

In some embodiments, the side surface of the temperature equalizing plate 42 facing the light-emitting component 200 includes the sheltered area 422 and the exposed area 423. The separation part 55 is supported on the sheltered area 422. The exposed area 423 is a flat area for guiding and dissipating heat. It can be understood that, since the exposed area 423 also forms the inner wall of the first heat dissipation channel 51, compared with the case where the exposed area 423 is uneven, the flat exposed area 423 can effectively reduce the air resistance when the first part of air flows in the first heat dissipation channel 51.

Specifically, the minimum distance between the exposed area 423 and the light-emitting component 200 may be greater than or equal to 6 mm, and the exposed area 423 is a flat area; or, the minimum distance between the exposed area 423 and the light-emitting component 200 may be less than 6 mm, and the exposed area 423 is a flat area; or, the minimum distance between the exposed area 423 and the light-emitting component 200 may be less than 6 mm, and the surface of the exposed area 423 is provided with an uneven structure, which is not limited in the present disclosure.

In the following, the flow diverting structure 42a being at least partially formed by some other parts of the skin treatment apparatus is described as an example.

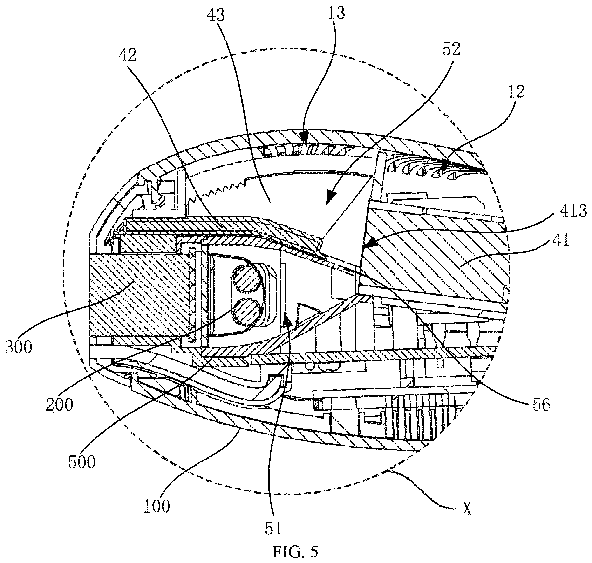

Illustratively, referring to FIG. 5, FIG. 5 is an enlarged diagram of part X in FIG. 2 showing another flow diverting structure. The skin treatment apparatus further includes the holder component 500 disposed inside the housing 100. The holder component 500 is configured to define the first heat dissipation channel 51 and the second heat dissipation channel 52. The first heat dissipation channel 51 is configured to allow the first part of air to flow through to dissipate heat from the light-emitting component 200. The second heat dissipation channel 52 is configured to allow the second part of air to flow through to dissipate heat from the cold compress component 300. The end of the holder component 500 close to the first air vent 413 is provided with a flow diverting part 56 to form the flow diverting structure 42a, so as to separate the air vent of the first heat dissipation channel 51 which is communicated with the fan 41 and the air vent of the second heat dissipation channel 52 which is communicated with the fan 41. By dissipating the heat from the light-emitting component 200 and the cold compress component 300 separately, the heat dissipation effects of the light-emitting component 200 and the cold compress component 300 are both ensured.

Illustratively, the light-emitting component 200 is at least partially disposed in the first heat dissipation channel 51, such that the first part of air can dissipate the heat from the light-emitting component 200 after flowing into the first heat dissipation channel 51.

The heat dissipation component 400 may include the temperature equalizing plate 42 and the heat dissipation sheet 43 mounted on the temperature equalizing plate 42. One end of the temperature equalizing plate 42 is thermally connected to the cold compress component 300. The temperature equalizing plate 42 and the heat dissipation sheet 43 are at least partially disposed in the second heat dissipation channel 52, such that the second part of air can dissipate the heat from the temperature equalizing plate 42 and the heat dissipation sheet 43 after flowing into the second heat dissipation channel 52, ultimately realizing the heat dissipation of the cold compress component 300.

In some embodiments, the light-emitting component 200 extends along the length direction of the first air vent 413. For example, in case the first air vent 413 is the air outlet of the fan 41, the first part of air flowing out of the first air vent 413 can be directly blown to more parts in the length direction of the light-emitting component 200, thereby improving the heat dissipation effect, and besides, the internal structure of the skin treatment apparatus becomes simpler and more compact.

In some embodiments, the first part of air and the second part of air may flow out from different regions of the first air vent 413 along the width direction. In other words, the near air vent section of the first heat dissipation channel 51 (if the first air vent 413 is the air outlet, then the near air vent section of the first heat dissipation channel 51 is its inlet section) and the near air vent section of the second heat dissipation channel 52 (if the first air vent 413 is the air outlet, then the near air vent section of the second heat dissipation channel 52 is its inlet section) are communicated with different regions of the first air vent 413 in the width direction.

For example, the light-emitting component 200 extends along the left-right direction, and the first air vent 413 also extends along the left-right direction. The inlet of the first heat dissipation channel 51 is communicated with the lower half area of the first air vent 413, and the inlet of the second heat dissipation channel 52 is communicated with the upper half area of the first air vent 413.

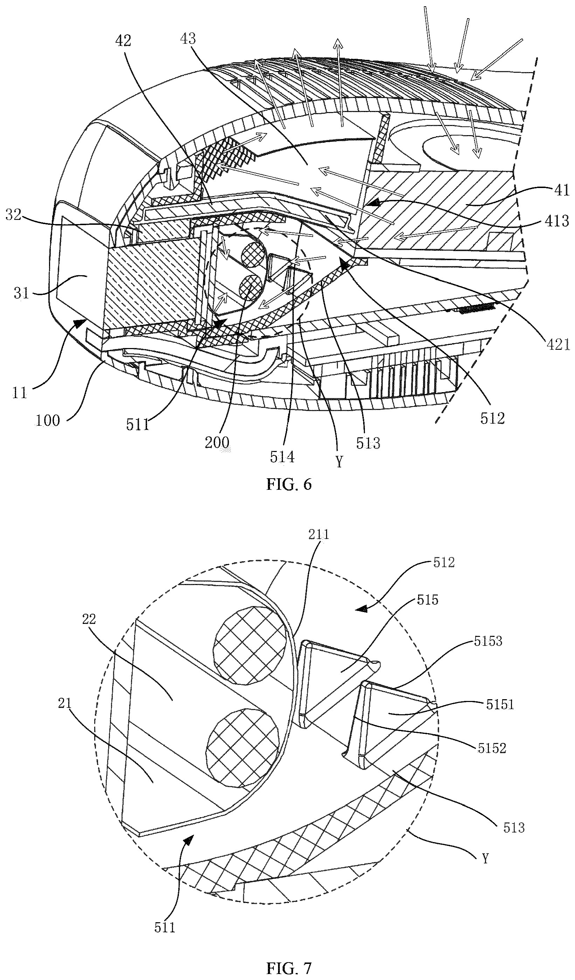

Referring to FIG. 6, FIG. 6 is another sectional diagram of the skin treatment apparatus of FIG. 1 along A-A direction. The first heat dissipation channel 51 includes a mounting section 511 and a flow guiding section 512 arranged along the direction of the light-emitting component 200 facing the first air vent 413. The light-emitting component 200 is at least partially mounted in the mounting section 511. One end of the flow guiding section 512 is located at the first air vent 413 to be communicated with the first air vent 413, and the other end of the flow guiding section 512 is communicated with the mounting section 511. This realizes the communication between the first heat dissipation channel 51 and the fan 41. For example, the first part of air flowing out from the first air vent 413 can directly flow to the mounting section 511 through the flow guiding section 512, to dissipate heat from the light-emitting component 200.

It can be understood that the near air vent section of the first heat dissipation channel 51 is defined in the flow guiding section 512.

It should be noted that the light-emitting component 200 being at least partially mounted in the mounting section 511 may be that the light-emitting component 200 is completely disposed in the mounting section 511. The light-emitting component 200 being at least partially mounted in the mounting section 511 may also be that the light-emitting component 200 is partially disposed in the mounting section 511. For example, two ends of the light-emitting component 200 are disposed through the inner wall of the mounting section 511, such that the light-emitting component 200 is partially disposed outside the mounting section 511; or, the light-emitting component 200 extends into the flow guiding section 512, which is not limited in the present disclosure.

In some embodiments, the cross-sectional area of at least partial region of the flow guiding section 512 gradually increases along the direction of the first air vent 413 facing the light-emitting component 200.

The flow guiding section 512 may be arranged as a diffuser structure. After a part of the air flowing out of the first air vent 413 flows into the flow guiding section 512, since the cross-sectional area of partial region in the flow guiding section 512 is gradually increased along the flow direction of the air, compared with the case where the cross-sectional area in the flow guiding section 512 is constant or gradually reduced along the flow direction of the air, the air resistance of the air flowing in the flow guiding section 512 is smaller. Therefore, the air flowing out of the first air vent 413 can smoothly flow into the flow guiding section 512, thereby improving the heat dissipation effect on the light-emitting component 200.

In some embodiments, the width of the flow guiding section 512 in the width direction of the first air vent 413 gradually increases. On one hand, it is beneficial for the cross-sectional area of the flow guiding section 512 to be gradually increased along the flow direction of the air, and on the other hand, the contact area between the first part of air and the light-emitting component 200 is increased. Therefore, the heat dissipation effect of the first part of air on the light-emitting component 200 is improved.

In some embodiments, the inner wall of the flow guiding section 512 includes a first inner side wall 513 facing the flow dividing structure 42a and a second inner side wall 514 facing the first inner side wall 513. Along the direction of the first air vent 413 facing the light-emitting component 200, at least one of the first inner side wall 513 and the second inner side wall 514 is inclined away from the other one, to allow the distance between the first inner side wall 513 and the second inner side wall 514 to be gradually increased.

Specifically, the first inner side wall 513 and the second inner side wall 514 may be inclined away from each other, or one of the first inner side wall 513 and the second inner side wall 514 may be inclined away from the other one, which is not limited in the present disclosure.

It can be understood that the second inner side wall 514 may be defined by the flow diverting structure 42a such as the temperature equalizing plate 42 and/or the flow diverting part 56 of the holder component 500, or may be defined by the flow diverting structure 42a and other parts, which is not limited in the present disclosure.

In some embodiments, the width of the end of the flow guiding section 512 close to the light-emitting component 200 is greater than or equal to the width of the light-emitting component 200 in the direction of the first inner sidewall 513 facing the second inner sidewall 514, such that the side surface of the light-emitting component 200 facing the first air vent 413 can be directly blown by the first part of air flowing out of the fan 41. This increases the heat dissipation area and thereby improving the heat dissipation effect on the light-emitting component 200.

Referring to FIG. 6 and FIG. 7, FIG. 7 is an enlarged diagram of part Y in FIG. 6. A first anti-turbulence structure 515 is provided in the flow guiding section 512 to limit a formation of turbulence in the flow guiding section 512. In this way, the first part of air can flow into the mounting section 511 from the flow guiding section 512 more smoothly for heat dissipation. In other words, the first part of air can flow faster in the flow guiding section 512, thereby improving the heat dissipation effect.

Illustratively, the light-emitting component 200 includes a light reflective member 21 and a light source 22 disposed in the light reflective member 21.

The outer wall of the light reflective member 21 includes a first curved surface protruding toward the flow guiding section 512. The first anti-turbulence structure 515 is configured to limit the formation of the turbulence between the first curved surface and the inner wall of the flow guiding section 512.

Specifically, after the first part of air is blown to the light-emitting component 200, it will be blocked by the first curved surface and partially return to the flow guiding section 512. In this case, the first anti-turbulence structure 515 can prevent the airflow blocked by the first curved surface and returning into the flow guiding section 512 and the airflow flowing from the fan 41 into the flow guiding section 512 from forming the turbulence, which allows the first part of air to flow more smoothly in the first heat dissipation channel 51, thereby improving the flow rate of the first part of air and the heat dissipation effect.

In some embodiments, the light source 22 may include a light tube. The light reflective member 21 includes an arc-shaped part 211 disposed around the light tube. The arc-shaped part 211 is at least partially located on the side of the light tube close to the flow guiding section 512. One end of the arc-shaped part 211 in the peripheral direction is adjacent to the first inner side wall 513, and the other end of the arc-shaped part 211 in the peripheral direction is adjacent to the second inner side wall 514. The first inner side wall 513 or the second inner side wall 514 is provided with the first anti-turbulence structure 515, or each of the first inner side wall 513 and the second inner side wall 514 is provided with the first anti-turbulence structure 515.

Therefore, the first anti-turbulence structure 515 can prevent the air returning from the outer surface of the arc-shaped part 211 to the first inner side wall 513 and/or the second inner side wall 514 from forming the turbulence.

Specifically, only the first inner side wall 513 is provided with the first anti-turbulence structure 515, or only the second inner side wall 514 is provided with the first anti-turbulence structure 515, or each of the first inner side wall 513 and the second inner side wall 514 is provided with the first anti-turbulence structure 515, which is not limited in the present disclosure.

In some embodiments, the first anti-turbulence structure 515 may be integrally formed with the corresponding first inner sidewall 513 or the second inner sidewall 514. Certainly, the first anti-turbulence structure 515 may be formed separately from the corresponding first inner sidewall 513 or the second inner sidewall 514. For example, the first anti-turbulence structure 515 may be detachably connected to the first inner sidewall 513 or the second inner sidewall 514. The present disclosure is not limited thereto.

In some embodiments, the first anti-turbulence structure 515 includes a flow guiding plate. The flow guiding plate extends along the direction of the first air vent 413 facing the light-emitting component 200, such that the first inner side wall 513 and/or the second inner side wall 514 provided with the flow guiding plate can guide the passing air by the flow guiding plate to limit the formation of the turbulence at the first inner side wall 513 and/or the second inner side wall 514.

There may be one flow guiding plate or a plurality of flow guiding plates. For example, the first anti-turbulence structure 515 may include a plurality of flow guiding plates, and the plurality of flow guiding plates are spaced apart along the length direction of the light-emitting component 200, such as the length direction of the light tube.

In some embodiments, the first anti-turbulence structure 515 includes the flow guiding plate. The flow guiding plate includes a first inclined surface 5151 and a second inclined surface 5152 facing each other in the thickness direction. Along the direction of the first air vent 413 facing the light-emitting component 200, the first inclined surface 5151 and the second inclined surface 5152 are inclined away from each other, such that the thickness of the end of the flow guiding plate close to the light-emitting component 200 is greater than the thickness of the end of the flow guiding plate facing away from the light-emitting component 200. This can also be simply understood as that the side of the flow guiding plate facing the first air vent 413 is sharper to form an air-breaking structure, which reduces the air resistance formed when the first part of air passes through the flow guiding plate, thereby improving the heat dissipation effect.

In some embodiments, the inner wall of the flow guiding section 512 includes a first flow guiding surface connected to the flow guiding plate. The flow guiding plate further includes a third inclined surface 5153. The third inclined surface 5153 is located on the side of the flow guiding plate facing away from the first flow guiding surface. Along the direction of the first air vent 413 facing the light-emitting component 200, the third inclined surface 5153 is inclined away from the first flow guiding surface, so that a height of the flow guiding plate protruding from the first flow guiding surface at an end of the flow guiding plate close to the light-emitting component is greater than a height of the flow guiding plate protruding from the first flow guiding surface at an end of the flow guiding plate away from the light-emitting component. This can also be simply understood as that the side of the flow guiding plate facing the first air vent 413 is sharper to form an air-breaking structure, which reduces the air resistance formed when the first part of air passes through the flow guiding plate, thereby improving the heat dissipation effect.

In some embodiments, the heat dissipation component 400 further includes the temperature equalizing plate 42 and the heat dissipation sheet 43 mounted on the temperature equalizing plate 42. One end of the temperature equalizing plate 42 is thermally connected to the cold compress component 300. The temperature equalizing plate 42 and the heat dissipation sheet 43 are at least partially disposed in the second heat dissipation channel 52. The end of the temperature equalizing plate 42 close to the first air vent 413 bends toward the side where the light-emitting component 200 is located, to form a deflection section 421. The deflection section 421 extends to the first air vent 413, such that the side of the deflection section 421 close to the light-emitting component 200, together with the holder component 500, defines at least part of the flow guiding section 512; and the side of the deflection section 421 facing away from the light-emitting component 200, together with the holder component 500, forms the inlet section of the second heat dissipation channel 52. In other words, the deflection section 421 extends to the first air vent 413, such that the side of the deflection section 421 close to the light-emitting component 200, together with the holder component 500, defines at least part of the flow guiding section 512; and the side of the deflection section 421 facing away from the light-emitting component 200, together with the holder component 500, forms the near air vent section of the second heat dissipation channel 52.

For example, the light-emitting component 200 may be located below the temperature equalizing plate 42, such that the lower surface of the temperature equalizing plate 42 forms the upper inner wall of the first heat dissipation channel 51. Besides, the end of the temperature equalizing plate 42 close to the first air vent 413 is deflected downwardly, such that the deflection section 421 of the temperature equalizing plate 42 forms at least part of the second inner side wall 514 of the flow guiding section 512. This simplifies the structure of the holder component 500, thereby reducing the molding difficulty of the holder component 500.

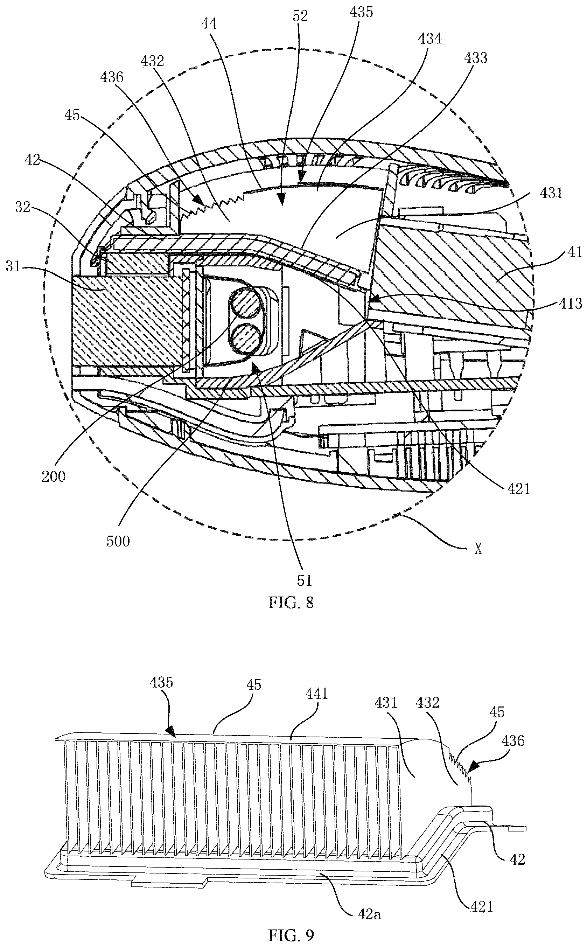

Referring to FIG. 8, FIG. 8 is another enlarged diagram of the part X in FIG. 2. In some embodiments, the heat dissipation sheet 43 may include a proximal heat dissipation section 431 and a distal heat dissipation section 432. The proximal heat dissipation section 431 is mounted on the deflection section 421. The distal heat dissipation section 432 is connected to the end of the proximal heat dissipation section 431 close to the light outlet area 11. The width of the proximal heat dissipation section 431 is greater than the width of the distal heat dissipation section 432, and the width of the heat dissipation sheet 43 is the distance from the side of the heat dissipation sheet 43 close to the temperature equalizing plate 42 to the side of the heat dissipation sheet 43 away from the temperature equalizing plate 42.

The heat dissipation sheet 43 includes a connection side edge 433 mounted on the temperature equalizing plate 42 and a free side edge 434 facing away from the temperature equalizing plate 42. The width of the heat dissipation sheet 43 is the distance from the connection side edge 433 of the heat dissipation sheet 43 to the free side edge 434 of the heat dissipation sheet 43.

Since the deflection section 421 is deflected downwardly, the space defined by the deflection section 421 that is deflected downwardly can be fully used, such that the proximal heat dissipation section 431 can be larger. This increases the contact area between the heat dissipation sheet 43 and the air, thereby improving the heat dissipation effect, and also ensures the overall structure of the skin treatment apparatus to be more compact and smaller.

For example, the heat dissipation sheet 43 includes the connection side edge 433 mounted on the temperature equalizing plate 42 and the free side edge 434 facing away from the temperature equalizing plate 42. Along the direction of the light-emitting component 200 facing the first air vent 413, the connection side edge 433 and the free side edge 434 of the proximal heat dissipation section 431 are inclined away from each other, to allow the width of the proximal heat dissipation section 431 to be gradually increased.

In some embodiments, the housing 100 includes a light outlet section extending from the junction of the proximal heat dissipation section 431 and the distal heat dissipation section 432 to the light outlet area 11. The cross-sectional area of the light outlet section gradually decreases along the direction of the light-emitting component 200 facing the light outlet area 11, such that the end surface of the housing on the side of the light outlet area 11 is smaller, which facilitates a user to accurately attach the light outlet area 11 to the user's skin to be treated. Correspondingly, the height of the distal heat dissipation section 432 protruding from the temperature equalizing plate 42 is also smaller, and the height of the proximal heat dissipation section 431 protruding from the temperature equalizing plate 42 is greater than the height of the distal heat dissipation section 432 protruding from the temperature equalizing plate 42.

In some embodiments, there are a plurality of heat dissipation sheets 43. Each heat dissipation sheet 43 includes the connection side edge 433 mounted on the temperature equalizing plate 42 and the free side edge 434 facing away from the temperature equalizing plate 42. The free side edges 434 of the plurality of heat dissipation sheets 43 define a free side surface. The free side surface includes an air blocking area 435 close to the first air vent 413 and a circulation area 436 close to the cold compress component 300. The heat dissipation component 400 further includes a blocking part 44. The blocking part 44 shields the air blocking area 435 to allow air between the adjacent heat dissipation sheets 43 flowing out or flowing into the circulation area 436 close to the cold compress component 300.

Therefore, the air in the second heat dissipation channel 52 is allowed to flow to a position closer to the cold compress component 300, which improves the heat dissipation effect on the cold compress component 300. Moreover, in case the end of the deflection section 421 close to the first air vent 413 is deflected downwardly, the blocking part 44 helps to better prevent the air flowing out of the first air vent 413 from being directly guided out from the heat dissipation section 431 by the deflection section 421.

In some embodiments, the blocking part 44 at least shields the free side edges 434 of the proximal heat dissipation section 431. Therefore, the blocking part 44 can prevent the air flowing out of the first air vent 413 from being directly guided out from the proximal heat dissipation section 431 by the deflection section 421.

In some embodiments, the length of the blocking part 44 accounts for more than one-third of the length of the heat dissipation sheet 43 in the direction of the first air vent 413 facing the light-emitting component 200, such that the air between the adjacent heat dissipation sheets 43 can flow out from the circulation area 436 close to the cold compress component 300.

Referring to FIG. 9, FIG. 9 is a schematic structural diagram of a first blocking part at the heat dissipation sheets of the skin treatment apparatus in FIG. 2. In some embodiments, the blocking part 44 may include an air baffle 441, which covers the free side edges 434 of the plurality of heat dissipation sheets 43.

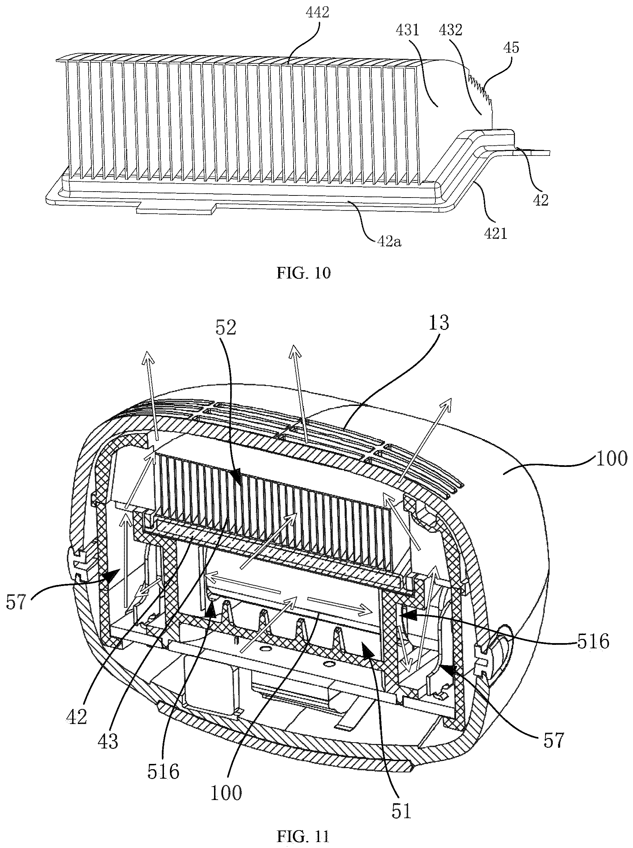

Referring to FIG. 10, FIG. 10 is a schematic structural diagram of a first blocking part at the heat dissipation sheets of the skin treatment apparatus in FIG. 2. In some embodiments, the blocking part 44 may include a plurality of folded edges 442. Each folded edge 442 is bendably connected to the free side edge 434 of one heat dissipation sheet 43. Therefore, in the manufacturing process, the folded edge 442 may be formed on the free side edge 434 of the heat dissipation sheet 43 directly by a bending or flanging process, which makes the manufacturing of the heat dissipation sheet 43 and the blocking part 44 simpler, thereby reducing the cost.

Specifically, the blocking part 44 may include one of the air baffle 441 and the folded edges 442, or may include both the air baffle 441 and the folded edges 442, which is not limited in the present disclosure.

The housing 100 is further defined with a second air vent 13 communicated with the outside of the housing 100. The second air vent 13 is located on the side of the heat dissipation sheet 43 facing away from the temperature equalizing plate 42. The second air vent 13 is disposed corresponding to the blocking part 44, and is communicated with the circulation area 436.

For example, in case the first air vent 413 is the air outlet of the fan 41, the second part of air flowing out of the first air vent 413 passes through the heat dissipation sheets 43 and is directly discharged through the second air vent 13, which makes the heat dissipation path of the second heat dissipation channel 52 shorter and the air flow faster. Moreover, since the second air vent 13 is disposed corresponding to the blocking part 44, which means that the second air vent 13 is farther away from the light outlet area 11, the hot air discharged from the second air vent 13 can be prevented from being directly blown onto the skin to be treated adjacent to the light outlet area 11, thereby improving the use experience of the skin treatment apparatus.

In some embodiments, a second anti-turbulence structure 45 is provided on the side of the heat dissipation sheet 43 away from the temperature equalizing plate 42 to limit a formation of turbulence on the side of the heat dissipation sheet 43 away from the temperature averaging plate 42.

For example, the second anti-turbulence structure 45 is located in the circulation area 436 to prevent the air flowing out of the circulation area 436 from forming the turbulence.

It can be understood that, since the height of the heat dissipation sheet 43 gradually decreases from the proximal heat dissipation section 431 to the distal heat dissipation section 432, or in other words, the space between the heat dissipation sheet 43 gradually decreases, and the blocking part 44 makes the second part of air flow to the distal heat dissipation section 432 and then flow out, it is prone to form the turbulence in the circulation area 436 at the distal heat dissipation section 432. Therefore, the second anti-turbulence structure 45 helps the air flowing out of the circulation area 436 to flow more smoothly, thereby improving the heat dissipation effect.

In some embodiments, each heat dissipation sheet 43 is provided with the second anti-turbulence structure 45. By providing more second anti-turbulence structures 45, the heat dissipation effect can be enhanced. Certainly, in some other embodiments, only one or part of the heat dissipation sheets 43 is provided with the second anti-turbulence structure 45, which is not limited in the present disclosure.

In some embodiments, the second anti-turbulence structure 45 may include a plurality of tooth parts, which are protruded on the side of the heat dissipation sheet 43 facing away from the temperature equalizing plate 42. Compared with the case where the side of the heat dissipation sheets 43 away from the temperature equalizing plate 42 is flat, the plurality of tooth parts can effectively limit the formation of the turbulence in the circulation area 436.

In some embodiments, the second anti-turbulence structure 45 may include a plurality of tooth parts, and the plurality of tooth parts are triangular. Certainly, in some other embodiments, the plurality of tooth parts may have other shapes, which is not limited in the present disclosure.

In some embodiments, the second anti-turbulence structure 45 may be integrally formed with the heat dissipation sheet 43. Certainly, the second anti-turbulence structure 45 may be formed separately from the heat dissipation sheet 43. For example, the second anti-turbulence structure 45 may be detachably connected to the heat dissipation sheet 43. The present disclosure is not limited thereto.

In some embodiments, the housing 100 is further provided with the second air vent 13 communicated with the outside of the housing 100. The second air vent 13 is located on the side of the heat dissipation sheet 43 facing away from the temperature equalizing plate 42. One end of the first heat dissipation channel 51 away from the first air vent 413 is communicated with the second air vent 13 so as to be communicated with the outside of the housing 100. The end of the second heat dissipation channel 52 away from the first air vent 413 is communicated with the second air vent 13 so as to be communicated with the outside of the housing 100.

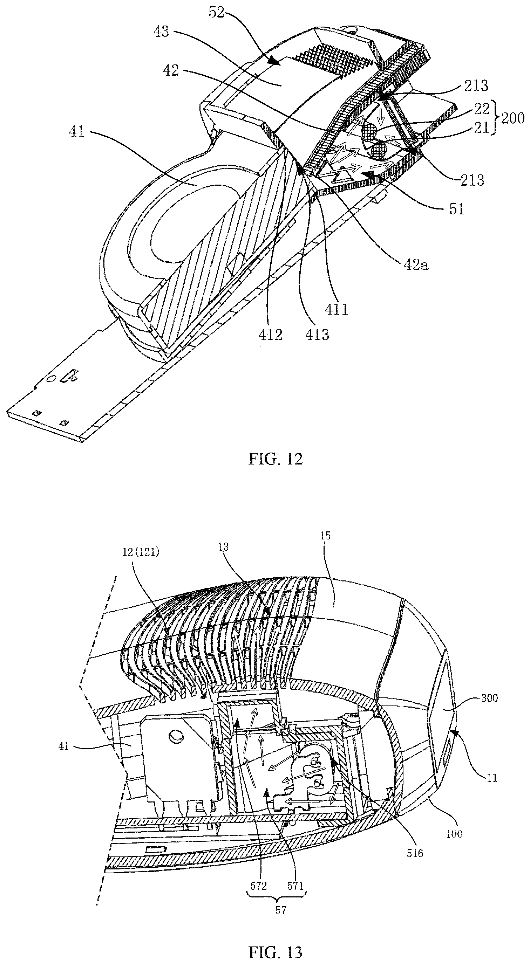

Referring to FIG. 11 and FIG. 12, FIG. 11 is a sectional diagram of the skin treatment apparatus in FIG. 2 along B-B direction, and FIG. 12 is a sectional diagram of the heat dissipation component and the light-emitting component of FIG. 2. In some embodiments, the housing 100 is further provided with the second air vent 13 communicated with the outside of the housing 100. The second air vent 13 is located on the side of the heat dissipation sheet 43 facing away from the temperature equalizing plate 42. The inner wall of the housing 100 is further defined with a third heat dissipation channel 57. One end of the third heat dissipation channel 57 is communicated with the end of the first heat dissipation channel 51 away from the first air vent 413, and the other end of the third heat dissipation channel 57 and the end of the second heat dissipation channel 52 away from the first air vent 413 are both communicated with the second air vent 13 and form a Bernoulli structure.

For example, in case the first air vent 413 is the air outlet of the fan 41, when the fan 41 is running, the pressure is lower at the outlet of one of the third heat dissipation channel 57 and the second heat dissipation channel 52 with a faster flow rate, so that the air in the other air channel is taken out or sucked out, which increases the flow rate in the other air channel, thereby improving the overall heat dissipation effect of the skin treatment apparatus.

For example, when the fan 41 is running, the flow rate of the air in the second heat dissipation channel 52 can be greater than the flow velocity of the air in the third heat dissipation channel 57. This allows the air in the second heat dissipation channel 52 to take out the air in the third heat dissipation channel 57, thereby allowing the air in the second heat dissipation channel 52 to indirectly take out the air in the first heat dissipation channel 51.

In some embodiments, the outlet at the end of the third heat dissipation channel 57 close to the second air vent 13 is defined in the inner wall of the outlet end of the second heat dissipation channel 52, which facilitates the air in the second heat dissipation channel 52 to take out the air in the third heat dissipation channel 57.

In some embodiments, the second heat dissipation channel 52 extends from the first air vent 413 to the second air vent 13. The heat dissipation sheet 43 is disposed in the second heat dissipation channel 52, and the heat dissipation sheet 43 is adjacent to both the first air vent 413 and the second air vent 13, so the second heat dissipation channel 52 is relatively short. The first heat dissipation channel 51 is located on the side of the temperature equalizing plate 42 facing away from the second air vent 13, so that the total length of the flow path of the first part of air in the first heat dissipation channel 51 and the third heat dissipation channel 57 is greater than the total length of the flow path of the second part of air in the second heat dissipation channel 52.

Referring to FIG. 13, FIG. 13 is another sectional diagram of the skin treatment apparatus of FIG. 1. The third heat dissipation channel 57 may include a diffuser cavity 571 and a communication section 572. The diffuser cavity 571 is communicated with the mounting section 511 through the mounting port 516 (that is, the diffuser cavity 571 is communicated with the mounting section 511 through the gap air vent 212 and the end air vent 214 described below). One end of the diffuser cavity 571 extends away from the light outlet area 11. The communication section 572 is located on the side of the diffuser cavity 571 close to the second air vent 13. One end of the communication section 572 is communicated with the end of the diffuser cavity 571 away from the light outlet area 11, and the other end of the communication section 572 is communicated with the inner wall of the end of the second heat dissipation channel 52 close to the second air vent 13, so that the first heat dissipation channel 51 and the second heat dissipation channel 52 are both communicated with the second air vent 13 and form a Bernoulli structure at the junction.

By providing the diffuser cavity 571, the air after passing through the light tube and the light reflective member 21 passes through the mounting opening 516 (namely the gap air outlet 212 and the end air outlet 214 described below) and enters a larger space, so as to reduce the air resistance. This allows the air after passing through the light tube and the light reflective member 21 to enter the diffuser cavity 571 through the gap air outlet 212 and the end air outlet 214 more smoothly, thereby improving the heat dissipation efficiency.

In some embodiments, the cross-sectional area of the diffuser cavity 571 may be gradually reduced along the direction away from the light outlet area 11, which allows the air in the diffuser cavity 571 to flow faster, thereby improving the heat dissipation effect on the light-emitting component 200.

Correspondingly, a circuit board 61 may form part of the inner wall of the diffuser cavity 571 or may be partially disposed in the diffuser cavity 571, to allow a conductive holder 62 to be disposed in the diffuser cavity 571.

Since the total length of the first part of air flowing in the first heat dissipation channel 51 and the second heat dissipation channel 52 is relatively large, the first part of air flows relatively slow. This allows the air in the second heat dissipation channel 52 to take out the air in the third heat dissipation channel 57, ultimately allowing the air in the second heat dissipation channel 52 to indirectly take out the air in the first heat dissipation channel 51.

Certainly, in case the height of the heat dissipation sheet 43 gradually decreases from the proximal heat dissipation section 431 to the distal heat dissipation section 432 in the second heat dissipation channel 52, when the air intake amount of the second heat dissipation channel 52 is constant, the air velocity at the outlet of the second heat dissipation channel 52 is correspondingly faster. This allows the air in the second heat dissipation channel 52 to take out the air in the third heat dissipation channel 57, ultimately allowing the air in the second heat dissipation channel 52 to indirectly take out the air in the first heat dissipation channel 51.

In some embodiments, the air intake amount at the inlet of the first heat dissipation channel 51 is less than the air intake amount at the inlet of the second heat dissipation channel 52. In other words, the air intake amount at the end of the first heat dissipation channel 51 communicated with the first air vent 413 is less than the air intake amount at the end of the second heat dissipation channel 52 communicated with the first air vent 413. Therefore, the air velocity at the outlet of the second heat dissipation channel 52 is greater. This allows the air in the second heat dissipation channel 52 to take out the air in the third heat dissipation channel 57, ultimately allowing the air in the second heat dissipation channel 52 to indirectly take out the air in the first heat dissipation channel 51.

More importantly, in order to ensure the cold compress effect of the cold compress component 300 and thereby realizing the freezing cold compress effect, the heat generated by the refrigeration sheet 32 needs to be discharged timely and quickly. Therefore, sufficient air volume and air velocity are required in the second heat dissipation channel 52, such that the heat generated by the refrigeration sheet 32 can be taken away by the heat dissipation sheets 43 and the temperature equalizing plate 42 timely and quickly. That is to say, in the present disclosure, by allowing the air intake amount at the inlet of the first heat dissipation channel 51 to be smaller than that at the inlet of the second heat dissipation channel 52, the air volume of the fan is reasonably distributed, realizing that the heat generated by the refrigeration sheet 32 is taken away by the heat dissipation sheets 43 and the temperature equalizing plate 42 timely and quickly. This improves the cold compress effect of the cold application component 300, thereby realizing the freezing cold compress. For example, the cold compress temperature of the cold compress component 300 can be rapidly reduced to zero degree or below zero degree (such as minus 5 degrees).

For example, the end of the first heat dissipation channel 51 close to the first air vent 413 is the air inlet of the first heat dissipation channel 51, and the end of the second heat dissipation channel 52 close to the first air vent 413 is the air inlet of the second heat dissipation channel 52. The size of the first air vent 413 corresponding to the air inlet of the first heat dissipation channel 51 is less than the size of the first air vent 413 corresponding to the air inlet of the second heat dissipation channel 52, such that the air intake amount at the air inlet of the first heat dissipation channel 51 is less than the air intake amount at the air inlet of the second heat dissipation channel 52.

Specifically, the first air vent 413 includes a first side edge 411 close to the first heat dissipation channel 51 and a second side edge 412 close to the second heat dissipation channel 52. The first side edge 411 faces the second side edge 412. A distance from the flow diverting structure 42a to the first side edge 411 is less than a distance from the flow diverting structure 42a to the second side edge 412, or in other words, a distance from the end of the deflection section 421 close to the first air vent 413 to the first side edge 411 is less than a distance from the end of the deflection section 421 close to the first air vent 413 to the second side edge 412, such that the air intake amount at the air inlet of the first heat dissipation channel 51 is less than the air intake amount at the air inlet of the second heat dissipation channel 52.

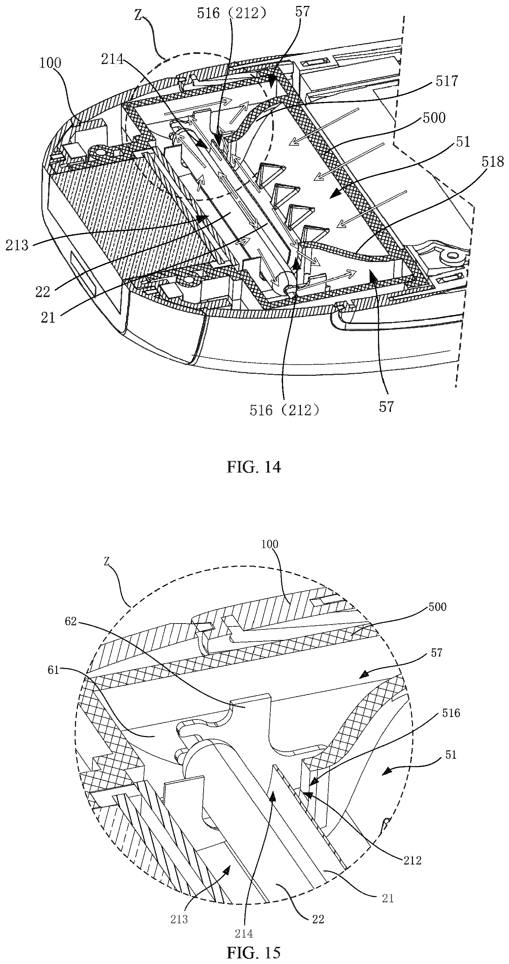

Referring to FIG. 12 to FIG. 15, FIG. 14 is a sectional diagram of the skin treatment apparatus of FIG. 2 along C-C direction, and FIG. 15 is an enlarged diagram of part Z in FIG. 14. In some embodiments, the inner wall of the first heat dissipation channel 51 is defined with a mounting opening 516. The light-emitting component 200 is mounted in the first heat dissipation channel 51 through the mounting opening 516, and defines the gap air vent 212 with the inner wall of the mounting opening 516. The light-emitting component 200 includes the light reflective member 21 and the light source 22. The light source 22 is disposed in the light reflective member 21. A side air vent 213 is defined in the peripheral side of the light reflective member 21. The side air vent 213 is communicated with the first heat dissipation channel 51. An end of the light reflective member 21 is defined with the end air vent 214.

For example, in case the first air vent 413 is the air outlet of the fan 41, a part of the air in the first heat dissipation channel 51 can be directly blown to the surface of the light reflective member 21, and the other part of the air can be blown into the interior of the light reflective member 21. This increases the heat dissipation area of the light-emitting component 200, thereby improving the heat dissipation effect on the light-emitting component 200.

In some embodiments, the mounting port 516 is communicated with the end of the third heat dissipation channel 57 close to the first heat dissipation channel 51, and the end of the third heat dissipation channel 57 away from the first heat dissipation channel 51 is communicated with the end of the second heat dissipation channel 52 away from the first air vent 413. The end air vent 214 is communicated with the mounting port 516 or the third heat dissipation channel 57.

For example, in case the first air vent 413 is the air outlet of the fan 41, a part of the air in the first heat dissipation channel 51 is directly blown to the surface of the light reflective member 21, and discharged successively through the gap air vent 212, the outlet of the third heat dissipation channel, the outlet of the second heat dissipation channel, and the second air vent 13; and the other part of the air in the first heat dissipation channel 51 is directly blown into the interior of the light reflective member 21 through the side air vent 213, and discharged successively through the end air vent 214, the outlet of the third heat dissipation channel 57, the outlet of the second heat dissipation channel 52, and the second air vent 13.

In some embodiments, the end air vent 214 is adjacent to the gap air vent 212 to form a Bernoulli structure.

For example, the light source 22 includes a light tube. The light reflective member 21 is disposed on the outer peripheral side of the light tube. The end of the light reflective member 21 is disposed through the mounting opening 516 along the length direction of the light tube, and the gap air vent 212 is defined by the outer wall of the light reflective member 21 and the inner wall of the mounting opening 516. The end air vent 214 is defined between the inner wall of the light reflective member 21 and the light tube along the length direction of the light tube, such that the gap air vent 212 and the end air vent 214 form the Bernoulli structure.

For example, in case the first air vent 413 is the air outlet of the fan 41, when the fan 41 is running, the pressure is lower at one of the end air vent 214 and the gap air vent 212 with a faster flow rate, so that the air in the air channel corresponding to the other vent is taken out or sucked out, thereby improving the overall heat dissipation effect of the skin treatment apparatus.

Specifically, since the flow path of the air flowing into the light reflective member 21 is more tortuous and longer, when the fan 41 operates, the flow velocity of the air flowing from the outside of the light reflective member 21 to the gap air outlet 212 is greater than the flow velocity of the air at the end air outlet 214, so that the air inside the light reflective member 21 is taken out.

Therefore, by forming another Bernoulli structure at the outlet of the second heat dissipation channel 52 and the outlet of the third heat dissipation channel 57, the skin treatment apparatus in the embodiments of the present disclosure can improve the flow velocity of the air inside the light reflective member 21 with the dual Bernoulli structures, thereby improving the heat dissipation effect.

In some embodiments, there may be one gap air vent 212 or a plurality of gap air vents 212. For example, at least one protrusion may be formed on the inner wall of the mounting opening 516, and the at least one protrusion abuts against the outer wall of the light reflective member 21, so as to form the plurality of gap air vents 212 or at least two gap air vents 212 between the inner wall of the mounting opening 516 and the outer wall of the light reflective member 21.

In some embodiments, the light outlet area 11 and the first air vent 413 are located on different sides in the peripheral direction of the light tube and face each other.

For example, the light outlet area 11 may be located on the front side of the light tube, and the first air vent 413 may be located on the rear side of the light tube.

In some embodiments, two side edges of the light reflective member 21 in the peripheral direction define an opening facing the light outlet area 11, so as to gather the light generated by the light tube to the light outlet area 11. At least one side edge of the light reflective member 21 in the peripheral direction is defined with the side air vent 213.

For example, two side edges of the reflective member 21 may face forward, so as to form a forward opening. In this case, each of the upper side edge and the lower side edge on the front side of the light reflective member 21 may be defined with the side air vent 213.

Specifically, the light reflective member 21 may be a reflective cup.

In case the flow guiding section 512 includes the first inner side wall 513 and the second inner side wall 514, one side edge of the light reflective member 21 in the peripheral direction is adjacent to the first inner side wall 513, so that the first inner side wall 513 guides a part of the air to the adjacent side air vent 213, and the other side edge of the reflective member 21 in the peripheral direction is adjacent to the second inner side wall 514, so that the second inner side wall 514 guides a part of the air to the adjacent side air vent 213.

In some embodiments, at least one end of the inner wall of the first heat dissipation channel 51 in the length direction of the light tube is defined with the mounting opening 516. The mounting openings 516 located on different sides in the length direction of the light tube are communicated to the second heat dissipation channel 52 through different third heat dissipation channels 57.

For example, the length direction of the light tube is the left-right direction. In this case, each of the left inner side wall and the right inner side wall of the first heat dissipation channel 51 may be defined with one mounting opening 516, and each mounting opening 516 is matched with one third heat dissipation channel 57.

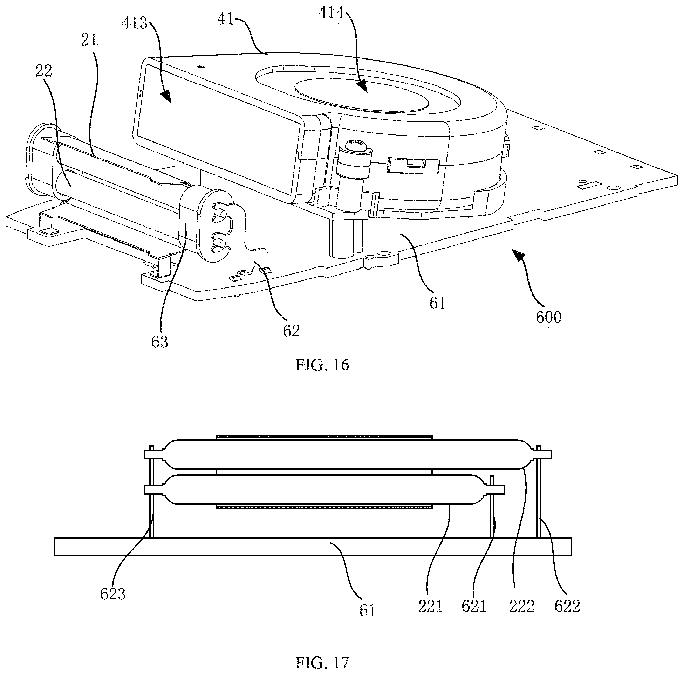

Referring to FIG. 16, FIG. 16 is a schematic diagram of a first circuit board component of the skin treatment apparatus in FIG. 2. In some embodiments, the skin treatment apparatus further includes a circuit board component 600, which includes the circuit board 61 and the conductive holder 62. The circuit board 61 is disposed in the housing 100. The conductive holder 62 is mounted on the circuit board 61.

Correspondingly, the light-emitting component 200 includes the light source 22. The light source 22 is mounted on the conductive holder 62, so as to be supported on the conductive holder 62 and electrically connected to the circuit board 61.

For example, the light-emitting component 200 may include a light tube. The end of the light tube is connected to the conductive holder 62, such that the light tube is supported on the circuit board 61 and is electrically connected to the circuit board 61.

In some embodiments, the circuit board 61 and the holder component 500 define the third heat dissipation channel 57, and the conductive holder 62 is located in the third heat dissipation channel 57. In this way, the third heat dissipation channel 57 is configured for mounting and fixing the light tube, thereby making the overall structure of the skin treatment apparatus more compact.

In some embodiments, there are at least two light sources 22, such as at least two light tubes. The at least two light sources 22 are spaced apart.

In this way, the care effect of the skin treatment apparatus can be improved by shining light from the plurality of light sources 22. For example, the plurality of light sources 22 may be switched on and off synchronously, to allow the light source component to operate at a higher maximum power for skin care, thereby improving the care effect of the skin treatment apparatus. For another example, each light source 22 may emit light alone or in combination with one another, to allow the light source component to have at least three light-emitting modes, which facilitates a user to select the appropriate light-emitting mode according to actual needs, thereby improving the care effect of the skin care apparatus.

For example, in case the plurality of light sources 22 can be switched on and off synchronously, the first ends of all the light sources 22 may be mounted on the same conductive holder 62 so as to be electrically connected to the circuit board 61, and the second ends of all the light sources 22 may be mounted on another conductive holder 62 so as to be electrically connected to the circuit board 61.

Specifically, the first end of the light source 22 such as the light tube is a positive electrode, and the second end of the light source 22 is a negative electrode; or, the first end of the light source 22 such as the light tube is a negative electrode, and the second end of the light source 22 is a positive electrode. This can also be understood as that the positive electrodes of all the light sources 22 are electrically connected to the circuit board 61 by the same conductive holder 62, and the negative electrodes of all the light sources 22 are electrically connected to the circuit board 61 by another conductive holder 62, which allows a more consistent transfer of current between the circuit board 61 and all the light sources 22. This improves the consistency of light emission of all the light sources 22, ultimately improving the care effect of the skin treatment apparatus.

The technical solution of the embodiments of the present disclosure is further described with an example in which different light sources 22 can emit light alone or in combination with one another.

Referring to FIG. 17, FIG. 17 is a schematic diagram of a second circuit board component of the skin treatment apparatus in FIG. 2. The at least two light sources 22 may include a first light source 221 and a second light source 222. At least three different light-emitting modes can be realized with the first light source 221 and the second light source 22. Certainly, the number of the light sources 22 may be three, four, five, or six, and the first light source 221 and the second light source 222 may be any two of the plurality of light sources 22, which is not limited in the present disclosure.

The conductive holder 62 includes a first conductive holder 621, a second conductive holder 622, and a third conductive holder 623. The positive electrodes of the first light source 221 and the second light source 222 are electrically connected to the third conductive holder 623. The negative electrode of the first light source 221 is electrically connected to the first conductive holder 621, and the negative electrode of the second light source 222 is electrically connected to the second conductive holder 622.

In this case, the third conductive holder 623 may be understood as a common positive electrode, which reduces the quantity of the conductive holders 62 required between the light sources 22 and the circuit board 61. This simplifies the structure of the skin treatment apparatus, thereby reducing the component difficulty and production cost of the skin treatment apparatus.

Moreover, it can also be understood that the circuit board 61, the third conductive holder 623, the first light source 221, and the first conductive holder 621 cooperatively form a first loop; the circuit board 61, the third conductive holder 623, the second light source 222, and the second conductive holder 622 cooperatively form a second loop. In this way, a switch may be disposed at a corresponding position of the first conductive holder 621, and another switch may be disposed at a corresponding position of the second conductive holder 622, so as to realize independent control of the first light source 221 and the second light source 222.

Alternatively, the conductive holder 62 may include a first conductive holder 621, a second conductive holder 622, and a third conductive holder 623. The negative electrodes of the first light source 221 and the second light source 222 are electrically connected to the third conductive holder 623. The positive electrode of the first light source 221 is electrically connected to the first conductive holder 621, and the positive electrode of the second light source 222 is electrically connected to the second conductive holder 622.

In this case, the third conductive holder 623 may be understood as a common negative electrode, which reduces the quantity of the conductive holders 62 required between the light sources 22 and the circuit board 61. This simplifies the structure of the skin treatment apparatus, thereby reducing the component difficulty and production cost of the skin treatment apparatus.

Moreover, it can also be understood that the circuit board 61, the third conductive holder 623, the first light source 221, and the first conductive holder 621 cooperatively form a first loop; the circuit board 61, the third conductive holder 623, the second light source 222, and the second conductive holder 622 cooperatively form a second loop. In this way, a switch may be disposed at a corresponding position of the first conductive holder 621, and another switch may be disposed at a corresponding position of the second conductive holder 622, so as to realize independent control of the first light source 221 and the second light source 222.

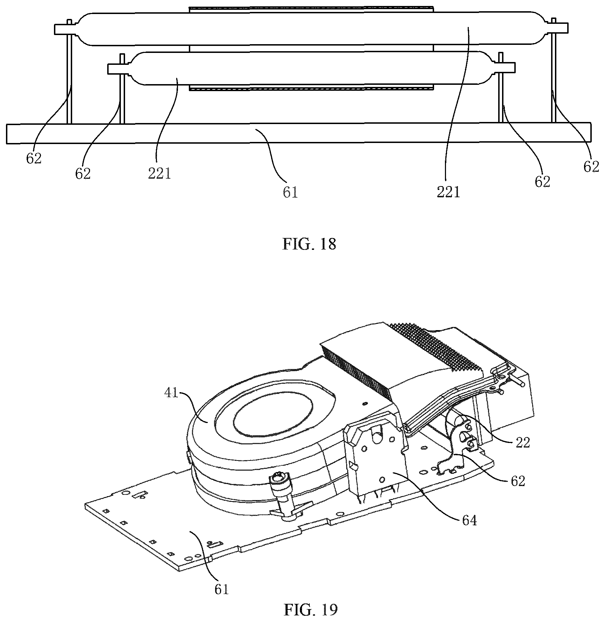

Referring to FIG. 18, FIG. 18 is a schematic diagram of a third circuit board component of the skin treatment apparatus in FIG. 2. Certainly, in some other embodiments, there are a plurality of the conductive holders 62. Different light sources 22 are mounted on the circuit board 61 by different conductive holders 62. In this way, in case any one of the conductive holders 62 falls off or is damaged, the remaining conductive holders 62 can still supply power to the corresponding light sources 22, which ensures that the other light sources 22 of the skin treatment apparatus can work normally. More importantly, this arrangement can increase the light output power (e.g., the hair removal power) and/or the diversity of the light output functions (e.g., the diversity of the hair removal functions). That is to say, by providing at least two light sources 22 that are mounted and fixed on the circuit board 61 by different conductive holders 62, multiple light sources 22 can be turned on at the same time when the hair removal power needs to be increased, and one of the light sources 22 can be turned on when the hair removal power does not need to be increased; or at least two light tubes of the at least two light sources 22 may emit light alternately, which increases the light output power, and at the same time, reduces or will not increase the loss of a single light source 22.

In some embodiments, the light-emitting component 200 further includes the light reflective member 21, which is disposed around the light tube. The light reflective member 21 is electrically connected to the circuit board 61, and the distance between the light reflective member 21 and the light tube is less than a preset distance, to allow the light reflective member 21 to excite the light tube.

For example, the light tube may be a xenon light tube, two ends or two electrodes of which are electrically connected to the circuit board 61 by the conductive holder 62. In this case, the circuit board 61 can excite or trigger the light tube by the light reflective member 21, such that the number of the parts of the skin treatment apparatus is reduced by multiplexing the light reflective member 21, thereby making the skin treatment apparatus more compact.

In some embodiments, the preset distance may be less than or equal to 2 mm. For example, the distance between the light reflective member 21 and the light tube is 2 mm, 1.4 mm, 0.5 mm, or 0 mm. Certainly, the distance between the light reflective member 21 and the light tube being 0 mm can be understood as that the light reflective member 21 directly abuts against the light tube.

In some embodiment, a wire (such as a filament) may be wound around the surface of the light tube, and the wire is electrically connected to the circuit board 61 for exciting the light tube, which is not limited in the present disclosure.

In some embodiments, the circuit board component 600 further includes a separation member 63, which is sleeved on at least one of the light tube and the conductive holder 62. The separation member 63 separates the reflective member 21 from the conductive holder 62. The separation member 63 is spaced from the mounting opening 516 to define an air outlet gap. Therefore, the separation member 63 helps to prevent an electricity leakage occurring between the light reflective member 21 for exciting the light tube and the conductive holder 62, thereby improving the reliability and safety of the skin treatment apparatus.

The separation member 63 may be made of an insulation material, such as plastic, rubber, or ceramic, which is not limited in the present disclosure.

As shown in FIG. 13, in some embodiments, the light-emitting component 200 includes a light tube, which is mounted in the mounting section 511. The inner wall of the flow guiding section 512 further includes a third inner side wall 517 and a fourth inner side wall 518. The third inner side wall 517 and the fourth inner side wall 518 are both connected between the first inner side wall 513 and the second inner side wall 514. The third inner side wall 517 and the fourth inner side wall 518 are located at different ends in the length direction of the light tube. Along the direction of the first air vent 413 facing the light-emitting component 200, at least one of the third inner side wall 517 and the fourth inner side wall 518 is inclined close to the other one, so as to guide the first part of air toward the light-emitting component 200.

It can be understood that, in an actual working process, the heat of the light tube is mainly concentrated in the middle of the light tube along the length direction. For example, in case the first air vent 413 is the air outlet of the fan 41, the first part of air can be concentrated in the middle of the light tube by the third inner side wall 517 and the fourth inner side wall 518, achieving a better heat dissipation effect.

For example, in the length direction of the light tube, the length of the end of the guide section 512 close to the light-emitting component 200 is less than the length of the light tube, such that the first part of air is concentrated in the middle of the light tube by the third inner side wall 517 and the fourth inner side wall 518.

In some embodiments, the holder component 500 is further defined with at least one third heat dissipation channel 57. The side of the third inner side wall 517 facing away from the fourth inner side wall 518 and/or the side of the fourth inner side wall 518 facing away from the third inner side wall 517 is defined with the third heat dissipation channel 57. The third heat dissipation channel 57 extends from the first inner side wall 513 toward the second inner side wall 514. One end of the third heat dissipation channel 57 is communicated with the outlet end of the first heat dissipation channel 51, and the other end of the third heat dissipation channel 57 is communicated with the second heat dissipation channel 52.

In this way, the escape space formed by the third inner side wall 517 and/or the fourth inner side wall 518 being inclined inward can be fully used to define the third heat dissipation channel 57, thereby making the overall structure of the skin treatment apparatus more compact.

Moreover, since the third inner side wall 517 and the fourth inner side wall 518 are inclined inward, the width of the third heat dissipation channel 57 may be gradually decreased along the direction of the light-emitting component 200 facing the first air vent 413, so as to pressurize and accelerate the air flow.

Referring to FIG. 19, FIG. 19 is schematic structural diagram of an IGBT member of the skin treatment apparatus in FIG. 2. In some embodiments, the circuit board component 600 may further include an IGBT member 64, which is mounted on the circuit board 61. An included angle between one side surface of the IGBT member 64 in the thickness direction and the circuit board 61 is greater than or equal to 45° and less than or equal to 90°.

For example, the included angle between the side surface of the IGBT member 64 in the thickness direction and the circuit board 61 may be 45°, 49°, 57.8°, 66.9°, 75.4°, 85°, or 90°, which is not limited in the present disclosure.

Compared with the case where one side surface of the IGBT member 64 in the thickness direction is closely attached to the circuit board 61, the embodiments of the present disclosure allow a larger gap to be formed between the IGBT member 64 and the circuit board 61. This increases the contact area between the IGBT member 64 and the air, thereby improving the heat dissipation effect on the IGBT member 64.

For example, the IGBT member 64 is longitudinally mounted on the circuit board 61, such that the included angle between the side surface of the IGBT member 64 in the thickness direction and the circuit board 61 is approximately equal to 90°.

In some embodiments, the fan 41 is configured to drive the air in the housing to flow out through the air outlet of the housing 100 along a preset path. The IGBT member 64 is at least partially located in the preset path. Therefore, the fan 41 can improve the heat dissipation effect on the IGBT member 64.

For example, the fan 41 may be mounted on the circuit board 61, and the IGBT member 64 is disposed close to the fan 41. The air inlet of the fan 41 can suck the air around it, such that the surrounding space of the fan 41 forms at least part of the preset path. Correspondingly, the IGBT member 64, which is disposed close to the fan 41, is thus at least partially mounted in the preset path.

In some embodiments, the circuit board 61 is disposed along the length direction of the housing 100. The circuit board 61 includes a first side edge and a second side edge extending along the length direction. The IGBT member 64 is located between the fan 41 and the first side edge, and is disposed adjacent to the first side edge.

The first side edge and the second side edge may be close to two side surfaces of the housing 100 in the width direction, or may be close to two side surfaces of the housing 100 in the thickness direction. Since the IGBT member 64 is disposed close to the first side edge, the space between the fan 41 and the first side edge can be reasonably used to mount the IGBT member 64, which facilitates the miniaturization design of the skin treatment apparatus.

In case the IGBT member 64 is disposed close to the fan 41, and the IGBT member 64 is longitudinally mounted, the IGBT member 64 can be substantially the same height as the fan 41, which reasonably uses the space in the thickness direction of the housing 100 on the side of the fan 41 facing the first side edge, thereby facilitating the miniaturization design of the skin treatment apparatus.

In some embodiments, one side surface of the IGBT member 64 in the thickness direction faces the fan 41. It can be understood that, since the size of the fan 41 is relatively large, the space occupied by the fan 41 in the length direction of the housing 100 is correspondingly large. Therefore, by arranging one side surface of the IGBT member 64 in the thickness direction toward the fan 41, the space in the length direction of the housing 100 on the side of the fan 41 facing the first side edge can be reasonably used, thereby facilitating the miniaturization design of the skin treatment apparatus.

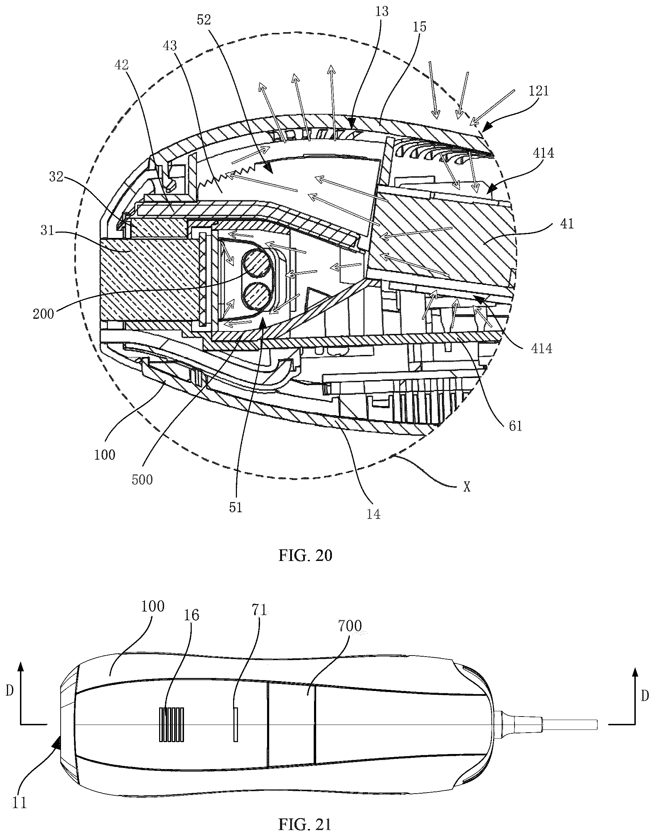

Referring to FIG. 20, FIG. 20 is a schematic structural diagram of FIG. 2 showing another fan of the skin treatment apparatus. In some embodiments, the fan 41 may be inclined with respect to the circuit board 61. Therefore, in case the fan 41 is mounted on the upper side of the circuit board 61, there is a larger heat dissipation gap between the bottom of the fan 41 and the circuit board 61, such that more areas of the upper surface of the circuit board 61 can be used for heat dissipation, wiring, or mounting electronic components, and the like, which facilitates the miniaturization design of the skin treatment apparatus.

In some embodiments, there are at least two air inlets of the fan 41, or in other words, at least two third air outlets 414. The side of the fan 41 facing away from the circuit board 61 is defined with a third air outlet 414, and the side of the fan 41 close to the circuit board 61 is defined with another third air outlet 414, so as to increase the air intake amount of the fan 41, thereby improving the heat dissipation effect.

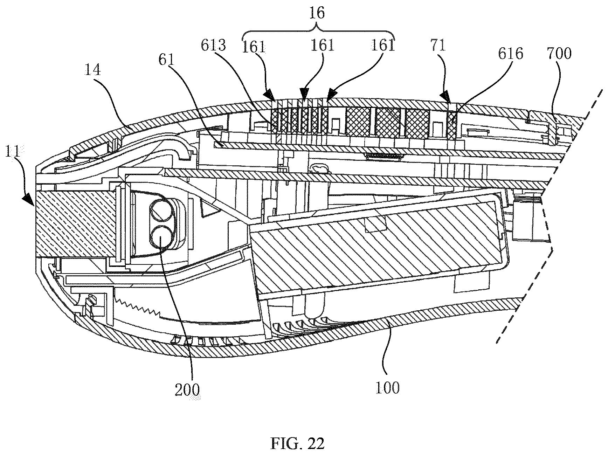

Referring to FIG. 21 and FIG. 22, FIG. 21 is a schematic structural diagram of the skin treatment apparatus in FIG. 1 from another perspective, and FIG. 22 is a sectional diagram of the skin treatment apparatus of FIG. 21 along D-D direction. In some embodiments, the housing 100 is further provided with a first display area 16. The first display area 16 has a plurality of indication forms. The skin treatment apparatus further includes a first light source component 613, which is disposed in the housing 100. The first light source component 613 is configured to generate light exiting from the first display area 16 for displaying a state of the skin treatment apparatus. The first light source component 613 is configured to be in an on or off state for displaying whether the skin treatment apparatus is attached to the skin, and is configured to display the indication forms in the first display area 16 for displaying gear position information of the skin treatment apparatus. This facilitates a user to observe the usage state of the skin treatment apparatus, thereby enhancing the user experience. Moreover, the number of the light source components of the skin treatment apparatus can be reduced, thereby reducing the cost of the skin treatment apparatus.

In some embodiments, the skin treatment apparatus may be further provided with a hair removal power control circuit, which is configured to control a power of the light-emitting component 200 or a gear position of the skin treatment apparatus. The hair removal power control circuit is electrically connected to the first light source component 613, so that the first light source component 613 can be configured to display the indication forms in in the first display area 16 for displaying the gear position information of the skin treatment apparatus.

In some embodiments, the skin treatment apparatus may further include a hair removal power control circuit, which is configured to control a power of the light-emitting component 200 or a gear position of the skin treatment apparatus. The hair removal power control circuit is electrically connected to the first light source component 613, so that the first light source component 613 can be configured to display the indication forms in in the first display area 16 for displaying the gear position information of the skin treatment apparatus.

In some embodiments, the skin treatment apparatus may further include an attachment detection circuit, which is configured to detect whether the light outlet area 11 is attached to the skin. For example, the attachment detection circuit includes a sensor configured to detect whether the light outlet area 11 is attached to the skin. The sensor may be an electrode sheet, a proximity optical sensor, a micro switch, an ultrasonic sensor, or the like, which is not limited in the present disclosure.

In some embodiments, the first display area 16 includes a plurality of first sub display areas 161. The first light source component 613 includes a plurality of light-emitting members. The first light source component 613 is configured to illuminate different numbers of the first sub-display areas 161 by controlling different numbers of the light-emitting members to emit light, so as to allow the first display area 16 to display different indication forms.

For example, the housing 100 may have a length direction, and the plurality of first sub-display areas 161 are arranged along the length direction of the housing 100. The plurality of light-emitting members are also arranged along the length direction of the housing 100, and each first sub-display area 161 is disposed corresponding to one or more light-emitting members. In this way, by controlling different light-emitting members in the length direction of the housing 100 to emit light, different numbers of the first sub-display areas 161 can be illuminated, so as to allow the first display area 16 to display different indication forms.

In some embodiments, at least two functions can be indicated by the display of the first display area 16. For example, whether the light outlet area 11 is attached to the skin, and the gear position information are indicated. That is to say, the first function (such as whether to attach to the skin) can be indicated by the on and off states of the first display area 16, and the second function (such as the gear position) can be indicated by the display area, the number, the proportion, or the like of the first display area 16.

Illustratively, when the light outlet area 11 is attached to the skin and the gear position information of the skin treatment apparatus is a first gear, one first sub-display area 161 may be illuminated, such that the first display area 16 displays the first indication form. When the light outlet area 11 is attached to the skin and the gear position information of the skin treatment apparatus is a second gear, two first sub display areas 161 may be illuminated, such that the first display area 16 displays the second indication form. When the light outlet area 11 is attached to the skin and the gear position information of the skin treatment apparatus is a third gear, three first sub display areas 161 may be illuminated, such that the first display area 16 displays the third indication form. However, when the light outlet area 11 is not attached to the skin, all the first sub-display areas 161 are not illuminated, that is, the first display area 16 is not illuminated, which indicates that the light outlet area 11 is not attached.

Alternatively, the plurality of first sub display areas 161 may be arranged along a circumferential direction or other curve direction, which is not limited in the present disclosure.

In some embodiments, the skin treatment apparatus may further include a control button 700, which is disposed on the housing 100 and is partially exposed out of the housing 100. The control button 700 is configured to control the skin treatment apparatus to work.

In some embodiments, the control button 700 and/or the area of the housing 100 close to the control button 700 is provided with a second display area 71, which is configured to display whether the skin treatment apparatus is connected to a power supply.

Therefore, when a user controls the skin treatment apparatus through the control button 700, it is convenient for he/she to observe the second display area 71, so as to confirm whether the skin treatment apparatus is connected to the power supply. This can avoid the user from mistakenly thinking that the skin treatment apparatus is damaged or has failed to complete the skin care treatment in case the skin treatment apparatus is not powered on. It can also be understood that, in case the second display area 71 is disposed on the control button 700, the number of the openings in the surface of the housing 100 can be reduced, thereby making the appearance of the housing 100 more beautiful.

In some embodiments, the skin treatment apparatus may further include a second light source component 616, which is disposed in the housing 100. The second light source component 616 is configured to generate light displayed in the second display area 71.

For example, the second light source component 616 may be a LED light, a tungsten light, or the like, which is not limited in the present disclosure.

In some embodiments, the housing 100 has a length direction, and the housing 100 includes a panel 14 extending along the length direction. The first display area 16 and the second display area 71 are spaced apart and are both disposed on the panel 14.

For example, the housing 100 may be elongated, and in this case, the first display area 16 and the second display area 71 are located on the same side of the housing 100 in the width or thickness direction, which is convenient for a user to observe the first display area 16 and the second display area 71 when holding one end of the housing 100 in the length direction.

It can be understood that the second display area 71 being disposed on the panel 14 may be that the second display area 71 is directly molded on the panel 14, or that the second display area 71 is disposed on the control button 700 which is disposed on the panel, and is thus indirectly disposed on the panel 14. The present disclosure is not limited thereto.

In some embodiments, the panel 14 may cover the first light source component 613 and/or the second light source component 616. The panel 14 is partially transparent to form the first display area 16 and/or the second display area 71.

For example, the panel 14 may include a light-transmitting plate. The light-transmitting plate includes a light-transmitting area and a light-shielding area. The light-shielding area is provided with a light-shielding structure such as a light-shielding layer. The light-transmitting area forms the first display area 16 and/or the second display area 71. In this way, the light emitted by the first light source component 613 and/or the second light source component 616 can be transmitted out through the light-transmitting area, and the light generated by the first light source component 613 and/or the second light source component 616 can be diffused randomly. Besides, the structure around the first light source component 613 and/or the second light source component 616 can be shielded, thereby making the housing 100 more aesthetically pleasing.

In some embodiments, the light-transmitting area of the panel 14 forms the first display area 16. The panel 14 may be further defined with a mounting hole. The control button 700 is disposed through the mounting hole, and the control button 700 is provided with the second display area 71.

Alternatively, the first display area 16 or the second display area 71 may be an opening structure defined in the surface of the housing 100, or each of the first display area 16 and the second display area 71 may be an opening structure defined in the surface of the housing 100, which is not limited in the present disclosure.

The description of each of the foregoing embodiment has a different emphasis, and the part not described in detail in a certain embodiment may refer to the related description in other embodiments.

In some embodiments, the housing 100 has a length direction, a width direction, and a thickness direction. The light outlet area 11 is located at one end of the housing 100, the length direction of the light tube is the same as the width direction of the housing 100, and the air inlet ends of the first heat dissipation channel 51 and the second heat dissipation channel 52 are spaced apart along the thickness direction of the housing 100. The housing 100 further includes a back plate extending along the length direction. The back plate of the housing 100 is defined with the housing air inlet and the housing air outlet. The housing air inlet is communicated with the air inlet of the fan 41, and the housing air inlet is communicated with the air outlet ends of the first heat dissipation channel 51 and the second heat dissipation channel 52.

The skin treatment apparatus provided in the embodiments of the present disclosure is described in detail above. The foregoing embodiments are intended only to assist in understanding the present disclosure. Those skilled in the art can understand that, based on the ideas of the present disclosure, there may be changes and variations to the embodiments and the application ranges. In summary, the contents of the specification shall not be construed as a limitation of the present disclosure.

## Claims

1. A skin treatment apparatus, comprising:
a housing (100) provided with a light outlet area (11);
a light-emitting component (200) arranged in the housing (100), the light-emitting component (200) being configured to generate light irradiated to a skin to be treated from the light outlet area (11);
a cold compress component (300) disposed at the light outlet area (11) for cooling the skin; and
a heat dissipation component (400), comprising:
a fan (41);
a temperature equalizing plate (42); and
a heat dissipation sheet (43) mounted on the temperature equalizing plate (42);
wherein the fan (41) is defined with a first air vent (413),
**characterized in that** one end of the temperature equalizing plate (42) is thermally connected to the cold compress component (300), and the other end of the temperature equalizing plate (42) is located at the first air vent (413); the fan (41) drives a first part of air to flow through a side of the temperature equalizing plate (42) facing away from the heat dissipation sheet (43) and flow through the light-emitting component (200) to dissipate heat from the light-emitting component (200), and drives a second part of air to flow through the heat dissipation sheet (43) to dissipate heat from the cold compress component (300).

2. The skin treatment apparatus according to claim 1, further comprising:
a holder component (500) arranged in the housing (100), the holder component (500) and the temperature equalizing plate (42) defining a first heat dissipation channel (51) for the first part of air to flow through; wherein,
the light-emitting component (200) is at least partially disposed in the first heat dissipation channel (51); and
the holder component (500) comprises:
a separation part (55), the separation part (55) separating the light-emitting component (200) from the temperature equalizing plate (42).

3. The skin treatment apparatus according to claim 2, wherein,
the separation part (55) is shorter than the temperature equalizing plate (42) along a direction of the light-emitting component (200) facing the first air vent (413), and the separation part (55) is supported on the temperature equalizing plate (42); and/or
a side surface of the temperature equalizing plate (42) facing the light-emitting component (200) comprises a sheltered area (422) and an exposed area (423), the separation part (55) is supported on the sheltered area (422), and a minimum distance between the exposed area (423) and the light-emitting component (200) is greater than or equal to 6 millimeters; and/or
a side surface of the temperature equalizing plate (42) facing the light-emitting component (200) comprises a sheltered area (422) and an exposed area (423), the separation part (55) is supported on the sheltered area (422), and the exposed area (423) is a flat area for guiding and dissipating heat; and/or
the skin treatment apparatus is a hair removal device or a skin rejuvenation device.

4. The skin treatment apparatus according to claim 1, further comprising:
a holder component (500) arranged in the housing (100), the holder component (500) and the temperature equalizing plate (42) defining a first heat dissipation channel (51) and a second heat dissipation channel (52); wherein,
the light-emitting component (200) is at least partially mounted in the first heat dissipation channel (51), one side of the temperature equalizing plate (42) and the holder component (500) define a near air vent section of the first heat dissipation channel (51), the near air vent section of the first heat dissipation channel (51) is communicated with the first air vent (413), and the fan (41) drives the first part of air to flow through the near air vent section of the first heat dissipation channel (51) to dissipate heat from the light-emitting component (200); and
the heat dissipation sheet (43) is disposed in the second heat dissipation channel (52), the other side of the temperature equalizing plate (42) and the holder component (500) define a near air vent section of the second heat dissipation channel, the near air vent section of the second heat dissipation channel (52) is communicated with the first air vent (413), and the fan (41) drives the second part of air to flow through the near air vent section of the second heat dissipation channel (52) to dissipate heat from the cold compress component (300).

5. The skin treatment apparatus according to claim 4, wherein the first heat dissipation channel (51) comprises:
a mounting section (511) and a flow guiding section (512) arranged along a direction of the light-emitting component (200) facing the first air vent (413); wherein,
the light-emitting component (200) is at least partially mounted in the mounting section (511);
one end of the flow guiding section (512) is located at the first air vent (413) to be communicated with the first air vent (413), and the other end of the flow guiding section (512) is communicated with the mounting section (511);
the near air vent section of the first heat dissipation channel (51) is defined in the flow guiding section (512); and
a cross-sectional area of at least partial region of the flow guiding section (512) gradually increases along a direction of the first air vent (413) facing the light-emitting component (200).

6. The skin treatment apparatus according to claim 5, wherein an inner wall of the flow guiding section (512) comprises:
a first inner side wall (513) facing the temperature equalizing plate (42); and
a second inner side wall (514) facing the first inner side wall (513);
wherein, along the direction of the first air vent (413) facing the light-emitting component (200), at least one of the first inner side wall (513) and the second inner side wall (514) is inclined away from the other one, to allow a distance between the first inner side wall (513) and the second inner side wall (514) to be gradually increased.

7. The skin treatment apparatus according to claim 6, wherein a first anti-turbulence structure (515) is provided in the flow guiding section (512) to limit a formation of turbulence in the flow guiding section (512).

8. The skin treatment apparatus according to claim 7, wherein the light-emitting component (200) comprises:
a light tube; and
a light reflective member (21), the light reflective member (21) comprising an arc-shaped part (211) disposed around the light tube, and the arc-shaped part (211) being at least partially located on a side of the light tube close to the flow guiding section (512);
wherein, one end of the arc-shaped part (211) in a peripheral direction is adjacent to the first inner side wall (513), and the other end of the arc-shaped part (211) in the peripheral direction is adjacent to the second inner side wall (514); and
the first inner side wall (513) or the second inner side wall (514) is provided with the first anti-turbulence structure (515), or each of the first inner side wall (513) and the second inner side wall (514) is provided with the first anti-turbulence structure (515);
and/or,
the first anti-turbulence structure (515) comprises a flow guiding plate, wherein,
the flow guiding plate extends along the direction of the first air vent (413) facing the light-emitting component (200); and/or
the flow guiding plate is one of a plurality of flow guiding plates, and the plurality of flow guiding plates are spaced apart along a length direction of the light-emitting component (200); and/or
the flow guiding plate comprises a first inclined surface (5151) and a second inclined surface (5152) facing each other along a thickness direction of the flow guiding plate; the first inclined surface (5151) and the second inclined surface (5152) are inclined away from each other along the direction of the first air vent (413) facing the light-emitting component (200), to allow a thickness of an end of the flow guiding plate close to the light-emitting component (200) to be greater than a thickness of an end of the flow guiding plate away from the light-emitting component (200); and/or
an inner wall of the flow guiding section (512) comprises a first flow guiding surface connected with the flow guiding plate, the flow guiding plate further comprises a third inclined surface (5153), the third inclined surface (5153) is located on a side of the flow guiding plate facing away from the first flow guiding surface; the third inclined surface (5153) is inclined away from the first flow guiding surface along the direction of the first air vent (413) facing the light-emitting component (200), to allow a height of the flow guiding plate protruding from the first flow guiding surface at an end of the flow guiding plate close to the light-emitting component (200) to be greater than a height of the flow guiding plate protruding from the first flow guiding surface at an end of the flow guiding plate away from the light-emitting component (200).

9. The skin treatment apparatus according to claim 5, wherein an end of the temperature equalizing plate (42) close to the first air vent (413) is deflected toward a side close to the light-emitting component (200), to form a deflection section (421);
wherein, the deflection section (421) extends to the first air vent (413), a side of the deflection section (421) close to the light-emitting component (200) and the holder component (500) define at least part of the flow guiding section (512), and a side of the deflection section (421) facing away from the light-emitting component (200) and the holder component (500) define the near air vent section of the second heat dissipation channel (52).

10. The skin treatment apparatus according to claim 9, wherein the heat dissipation sheet (43) comprises:
a proximal heat dissipation section (431) mounted on the deflection section (421); and
a distal heat dissipation section (432) connected to an end of the proximal heat dissipation section (431) close to the light outlet area (11);
wherein, a width of the proximal heat dissipation section (431) is greater than a width of the distal heat dissipation section (432), and a width of the heat dissipation sheet (43) is a distance from a side of the heat dissipation sheet (43) close to the temperature equalizing plate (42) to a side of the heat dissipation sheet (43) away from the temperature equalizing plate (42).

11. The skin treatment apparatus according to claim 10, wherein the heat dissipation sheet (43) comprises:
a connection side edge (433) mounted on the temperature equalizing plate; and
a free side edge (434) facing away from the temperature equalizing plate (42);
wherein, along the direction of the light-emitting component (200) facing the first air vent (413), the connection side edge (433) and the free side edge (434) of the proximal heat dissipation section (431) are inclined away from each other, to allow a width of the proximal heat dissipation section (431) to be gradually increased; and/or,
the housing (100) comprises:
a light outlet section extending from a junction of the proximal heat dissipation section (431) and the distal heat dissipation section (432) to the light outlet area (11);
wherein a cross-sectional area of the light outlet section gradually decreases along a direction of the light-emitting component (200) facing the light outlet area (11).

12. The skin treatment apparatus according to claim 10, wherein the heat dissipation sheet (43) is one of a plurality of heat dissipation sheets (43), each heat dissipation sheet (43) comprises the connection side edge (433) mounted on the temperature equalizing plate (42) and the free side edge (434) facing away from the temperature equalizing plate (42), the free side edges (434) of the plurality of heat dissipation sheets (43) define a free side surface, and the free side surface comprises an air blocking area (435) close to the first air vent (413) and a circulation area (436) close to the cold compress component (300);
wherein, the heat dissipation component (400) further comprises a blocking part (44), the blocking part (44) blocks the air blocking area (435), and air between adjacent heat dissipation sheets (43) flows out or flows into the circulation area (436) close to the cold compress component (300).

13. The skin treatment apparatus according to claim 5, wherein,
the housing (100) is further defined with a second air vent (13) communicated with an outside of the housing (100), and the second air vent (13) is located on a side of the heat dissipation sheet (43) facing away from the temperature equalizing plate (42); and
an inner wall of the housing (100) further defines a third heat dissipation channel (57), one end of the third heat dissipation channel (57) is communicated with an end of the first heat dissipation channel (51) away from the first air vent (413), and the other end of the third heat dissipation channel (57) and an end of the second heat dissipation channel (52) away from the first air vent (413) are both communicated with the second air vent (13) and form a Bernoulli structure at the junction.

14. The skin treatment apparatus according to claim 13, wherein an outlet at an end of the third heat dissipation channel (57) close to the second air vent (13) is defined in an inner wall at an end of the second heat dissipation channel (52) close to the second air vent (13);
and/or,
the second heat dissipation channel (52) extends from the first air vent (413) of the fan (41) to the second air vent (13) of the housing (100), the heat dissipation sheet (43) is disposed in the second heat dissipation channel (52), and the heat dissipation sheet (43) is adjacent to both the first air vent (413) and the second air vent (13); and
the first heat dissipation channel (51) is located on a side of the temperature equalizing plate (42) facing away from the second air vent (13), to allow a total length of a flow path of air in the first heat dissipation channel (51) and the third heat dissipation channel (57) to be greater than a total length of a flow path of air in the second heat dissipation channel (52);
and/or,
the skin treatment apparatus further comprises a circuit board component (600), the circuit board component (600) comprising:
a circuit board (61) disposed in the housing (100), the circuit board (61) and the holder component (500) defining the third heat dissipation channel (57); and
a conductive holder (62) mounted on the circuit board (61) and located in the third heat dissipation channel (57);
wherein, the light-emitting component (200) comprises a light tube, and an end of the light tube is connected with the conductive holder (62), to allow the light tube to be supported on the circuit board (61) and electrically connected with the circuit board (61).

15. The skin treatment apparatus according to claim 5, wherein an inner wall of the first heat dissipation channel (51) is defined with a mounting port (516), the light-emitting component is mounted in the first heat dissipation channel (51) through the mounting port (516), and the light-emitting component (200) and an inner wall of the mounting port (516) define a gap air vent (212); and
the light-emitting component (200) comprises a light reflective member (21) and a light source (22), the light source (22) is arranged in the light reflective member (21), a side air vent (213) is defined in a peripheral side of the light reflective member (21), the side air vent (213) is communicated with the first heat dissipation channel (51), and an end of the light reflective member (21) is defined with an end air vent (214).

## Patentansprüche

1. Hautbehandlungsvorrichtung, mit:
einem Gehäuse (100), das mit einem Lichtaustrittsbereich (11) versehen ist;
einer in dem Gehäuse (100) angeordneten Lichtemissionskomponente (200), die dazu eingerichtet ist, Licht zu erzeugen, das auf eine zu behandelnde Haut von dem Lichtaustrittsbereich (11) aus abgestrahlt wird;
einer an dem Lichtaustrittsbereich (11) angeordneten Kaltpresskomponente (300) zum Kühlen der Haut; und
einer Wärmeableitungskomponente (400), die einen Lüfter (41), eine Temperaturausgleichsplatte (42) und ein an der Temperaturausgleichsplatte (42) angebrachtes Wärmeableitungsblech (43) aufweist;
wobei der Lüfter (41) mit einer ersten Luftöffnung (413) definiert ist,
**dadurch gekennzeichnet,**
**dass** ein Ende der Temperaturausgleichsplatte (42) thermisch mit der Kaltpresskomponente (300) verbunden ist und das andere Ende der Temperaturausgleichsplatte (42) an der ersten Luftöffnung (413) angeordnet ist; wobei der Lüfter (41) einen ersten Luftteil dazu antreibt,
durch eine von dem Wärmeableitungsblech (43) abgewandte Seite der Temperaturausgleichsplatte (42) und durch die Lichtemissionskomponente (200) zu strömen, um Wärme von der Lichtemissionskomponente (200) abzuleiten, und einen zweiten Luftteil dazu antreibt, durch das Wärmeableitungsblech (43) zu strömen, um Wärme von der Kaltpresskomponente (300) abzuleiten.

2. Hautbehandlungsvorrichtung nach Anspruch 1, ferner mit
einer in dem Gehäuse (100) angeordneten Halterungskomponente (500), wobei die Halterungskomponente (500) und die Temperaturausgleichsplatte (42) einen ersten Wärmeableitungskanal (51) definieren, durch den der erste Luftteil strömt; wobei die Lichtemissionskomponente (200) zumindest teilweise in dem ersten Wärmeableitungskanal (51) angeordnet ist; und
wobei die Halterungskomponente (500) einen Trennabschnitt (55) aufweist, der die Lichtemissionskomponente (200) von der Temperaturausgleichsplatte (42) trennt.

3. Hautbehandlungsvorrichtung nach Anspruch 2,
wobei der Trennabschnitt (55) entlang einer Richtung der der ersten Luftöffnung (413) zugewandten Lichtemissionskomponente (200) kürzer als die Temperaturausgleichsplatte (42) ist und auf der Temperaturausgleichsplatte (42) abgestützt ist; und/oder wobei eine der Lichtemissionskomponente (200) zugewandte Seitenfläche der Temperaturausgleichsplatte (42) einen abgeschirmten Bereich (422) und einen freiliegenden Bereich (423) aufweist, wobei der Trennabschnitt (55) auf dem abgeschirmten Bereich (422) abgestützt ist und ein Mindestabstand zwischen dem freiliegenden Bereich (423) und der Lichtemissionskomponente (200) größer oder gleich 6 Millimeter ist; und/oder wobei eine der Lichtemissionskomponente (200) zugewandte Seitenfläche der Temperaturausgleichsplatte (42) einen abgeschirmten Bereich (422) und einen freiliegenden Bereich (423) aufweist, wobei der Trennabschnitt (55) auf dem abgeschirmten Bereich (422) abgestützt ist und der freiliegende Bereich (423) eine ebene Fläche zum Führen und Ableiten von Wärme ist; und/oder
wobei die Hautbehandlungsvorrichtung ein Haarentfernungsgerät oder ein Hautverjüngungsgerät ist.

4. Hautbehandlungsvorrichtung nach Anspruch 1, ferner mit
einer in dem Gehäuse (100) angeordneten Halterungskomponente (500), wobei die Halterungskomponente (500) und die Temperaturausgleichsplatte (42) einen ersten Wärmeableitungskanal (51) und einen zweiten Wärmeableitungskanal (52) definieren; wobei die Lichtemissionskomponente (200) zumindest teilweise in dem ersten Wärmeableitungskanal (51) angebracht ist, wobei eine Seite der Temperaturausgleichsplatte (42) und die Halterungskomponente (500) einen luftöffnungsnahen Abschnitt des ersten Wärmeableitungskanals (51) definieren, wobei der luftöffnungsnahe Abschnitt mit der ersten Luftöffnung (413) verbunden ist und der Lüfter (41) den ersten Luftteil durch den luftöffnungsnahen Abschnitt zur Ableitung von Wärme von der Lichtemissionskomponente (200) antreibt; und
wobei das Wärmeableitungsblech (43) in dem zweiten Wärmeableitungskanal (52) angeordnet ist, wobei die andere Seite der Temperaturausgleichsplatte (42) und die Halterungskomponente (500) einen luftöffnungsnahen Abschnitt des zweiten Wärmeableitungskanals (52) definieren, und wobei der luftöffnungsnahe Abschnitt mit der ersten Luftöffnung (413) verbunden ist und der Lüfter (41) den zweiten Luftteil durch diesen zur Ableitung von Wärme von der Kaltpresskomponente (300) antreibt.

5. Hautbehandlungsvorrichtung nach Anspruch 4, wobei der erste Wärmeableitungskanal (51)
einen Montageabschnitt (511) und einen Strömungsführungsabschnitt (512), die entlang einer Richtung der der ersten Luftöffnung (413) zugewandten Lichtemissionskomponente (200) angeordnet sind, aufweist;
wobei die Lichtemissionskomponente (200) zumindest teilweise in dem Montageabschnitt (511) angebracht ist;
wobei ein Ende des Strömungsführungsabschnitts (512) an der ersten Luftöffnung (413) angeordnet und mit dieser verbunden ist und das andere Ende mit dem Montageabschnitt (511) verbunden ist;
wobei der luftöffnungsnahe Abschnitt des ersten Wärmeableitungskanals (51) in dem Strömungsführungsabschnitt (512) definiert ist; und
wobei eine Querschnittsfläche zumindest eines Bereichs des Strömungsführungsabschnitts (512) entlang einer Richtung von der ersten Luftöffnung (413) zu der Lichtemissionskomponente (200) allmählich zunimmt.

6. Hautbehandlungsvorrichtung nach Anspruch 5,
wobei eine Innenwand des Strömungsführungsabschnitts (512)
eine erste innere Seitenwand (513), die der Temperaturausgleichsplatte (42) zugewandt ist und eine zweite innere Seitenwand (514), die der ersten inneren Seitenwand (513) zugewandt ist, aufweist; und
wobei entlang der Richtung von der ersten Luftöffnung (413) zu der Lichtemissionskomponente (200) wenigstens eine der ersten inneren Seitenwand (513) und die zweite innere Seitenwand (514) von der jeweils anderen weg geneigt ist, sodass ein Abstand zwischen der ersten inneren Seitenwand (513) und der zweiten inneren Seitenwand (514) allmählich zunimmt.

7. Hautbehandlungsvorrichtung nach Anspruch 6, wobei in dem Strömungsführungsabschnitt (512) eine erste Turbulenzverhinderungsstruktur (515) vorgesehen ist, um die Bildung von Turbulenzen in dem Strömungsführungsabschnitt (512) zu begrenzen.

8. Hautbehandlungsvorrichtung nach Anspruch 7,
wobei die Lichtemissionskomponente (200) eine Lichtröhre und ein Lichtreflexionselement (21) aufweist, wobei das Lichtreflexionselement (21) einen bogenförmigen Abschnitt (211), der um die Lichtröhre herum angeordnet ist, aufweist; wobei der bogenförmige Abschnitt (211) zumindest teilweise auf einer dem Strömungsführungsabschnitt (512) nahen Seite der Lichtröhre angeordnet ist;
wobei ein Ende des bogenförmigen Abschnitts (211) in Umfangsrichtung an die erste innere Seitenwand (513) angrenzt und das andere Ende in Umfangsrichtung an die zweite innere Seitenwand (514) angrenzt;
wobei die erste innere Seitenwand (513) oder die zweite innere Seitenwand (514) mit der ersten Turbulenzverhinderungsstruktur (515) versehen ist oder die erste innere Seitenwand (513) und die zweite innere Seitenwand (514) mit der ersten Turbulenzverhinderungsstruktur (515) versehen sind; und/oder
wobei die erste Turbulenzverhinderungsstruktur (515) eine Strömungsführungsplatte aufweist,
wobei die Strömungsführungsplatte sich entlang der Richtung von der ersten Luftöffnung (413) zu der Lichtemissionskomponente (200) erstreckt; und/oder
wobei die Strömungsführungsplatte eine von einer Mehrzahl von Strömungsführungsplatten ist, und die Mehrzahl von Strömungsführungsplatten entlang einer Längsrichtung der Lichtemissionskomponente (200) beabstandet angeordnet sind; und/oder wobei die Strömungsführungsplatte eine erste geneigte Fläche (5151) und eine zweite geneigte Fläche (5152) aufweist, die sich entlang einer Dickenrichtung der Strömungsführungsplatte gegenüberliegen, wobei sich erste geneigte Fläche (5151) und die zweite geneigte Fläche (5152) entlang der Richtung von der ersten Luftöffnung (413) zu der Lichtemissionskomponente (200) voneinander weg neigen, sodass die Dicke eines der Lichtemissionskomponente (200) nahen Endes der Strömungsführungsplatte größer ist als die Dicke eines von der Lichtemissionskomponente (200) entfernten Endes der Strömungsführungsplatte; und/oder
wobei die Innenwand des Strömungsführungsabschnitts (512) eine mit der Strömungsführungsplatte verbundene erste Strömungsführungsfläche aufweist und die Strömungsführungsplatte ferner eine dritte geneigte Fläche (5153) aufweist, wobei die dritte geneigte Fläche (5153) von der ersten Strömungsführungsfläche abgewandt ist und sich entlang der genannten Richtung von dieser weg neigt, sodass die Höhe des Vorsprungs der Strömungsführungsplatte an dem der Lichtemissionskomponente (200) nahen Ende größer ist als an dem entfernten Ende.

9. Hautbehandlungsvorrichtung nach Anspruch 5, wobei ein der ersten Luftöffnung (413) nahes Ende der Temperaturausgleichsplatte (42) zu einer der Lichtemissionskomponente (200) nahen Seite abgelenkt ist, um einen Ablenkabschnitt (421) zu bilden;
wobei sich der Ablenkabschnitt (421) zu der ersten Luftöffnung (413) erstreckt, eine der Lichtemissionskomponente (200) nahe Seite des Ablenkabschnitts (421) und die Halterungskomponente (500) zumindest einen Teil des Strömungsführungsabschnitts (512) definieren, und eine von der Lichtemissionskomponente (200) abgewandte Seite des Ablenkabschnitts (421) und die Halterungskomponente (500) den luftöffnungsnahen Abschnitt des zweiten Wärmeableitungskanals (52) definieren.

10. Hautbehandlungsvorrichtung nach Anspruch 9, wobei das Wärmeableitungsblech (43) einen proximalen Wärmeableitungsabschnitt (431), der an dem Ablenkabschnitt (421) angebracht ist; und einen distalen Wärmeableitungsabschnitt (432), der mit einem Ende des proximalen Wärmeableitungsabschnitts (431), das nahe dem Lichtaustrittsbereich (11) liegt, verbunden ist, aufweist;
wobei eine Breite des proximalen Wärmeableitungsabschnitts (431) größer ist als eine Breite des distalen Wärmeableitungsabschnitts (432), und eine Breite des Wärmeableitungsblechs (43) ein Abstand von einer Seite des Wärmeableitungsblechs (43), die nahe der Temperaturausgleichsplatte (42) liegt, zu einer Seite des Wärmeableitungsblechs (43), die von der Temperaturausgleichsplatte (42) abgewandt ist, ist.

11. Hautbehandlungsvorrichtung nach Anspruch 10, wobei das Wärmeableitungsblech (43):
eine Verbindungsseitenkante (433), die an der Temperaturausgleichsplatte angebracht ist; und eine freie Seitenkante (434), die von der Temperaturausgleichsplatte (42) abgewandt ist, aufweist;
wobei sich entlang der Richtung der Lichtemissionskomponente (200), die der ersten Luftöffnung (413) zugewandt ist, die Verbindungsseitenkante (433) und die freie Seitenkante (434) des proximalen Wärmeableitungsabschnitts (431) voneinander weg neigen, sodass eine Breite des proximalen Wärmeableitungsabschnitts (431) allmählich zunimmt; und/oder das Gehäuse (100) einen Lichtaustrittsabschnitt, der sich von einer Verbindungsstelle des proximalen Wärmeableitungsabschnitts (431) und des distalen Wärmeableitungsabschnitts (432) zu dem Lichtaustrittsbereich (11) erstreckt, aufweist;
wobei eine Querschnittsfläche des Lichtaustrittsabschnitts entlang einer Richtung der Lichtemissionskomponente (200), die dem Lichtaustrittsbereich (11) zugewandt ist, allmählich abnimmt.

12. Hautbehandlungsvorrichtung nach Anspruch 10, wobei das Wärmeableitungsblech (43) eines aus einer Mehrzahl von Wärmeableitungsblechen (43) ist,
jedes Wärmeableitungsblech (43) eine Verbindungsseitenkante (433), die an der Temperaturausgleichsplatte (42) montiert ist, und eine freie Seitenkante (434), die von der Temperaturausgleichsplatte (42) abgewandt ist, aufweist,
die freien Seitenkanten (434) der Mehrzahl von Wärmeableitungsblechen (43) eine freie Seitenfläche definieren, und
die freie Seitenfläche einen Luftsperrbereich (435), der nahe der ersten Luftöffnung (413) liegt, und einen Zirkulationsbereich (436), der nahe der Kaltpresskomponente (300) liegt, aufweist,
wobei die Wärmeableitungskomponente (400) ferner einen Blockierungsabschnitt (44) aufweist, wobei der Blockierungsabschnitt (44) den Luftblockierungsbereich (435) blockiert, und Luft zwischen benachbarten Wärmeableitungsblechen (43) aus dem der Kaltpresskomponente (300) nahen Zirkulationsbereich (436) herausströmt oder in den der Kaltpresskomponente (300) nahen Zirkulationsbereich (436) hineinströmt.

13. Hautbehandlungsvorrichtung nach Anspruch 5, wobei das Gehäuse (100) ferner eine zweite Luftöffnung (13), die mit der Außenseite des Gehäuses (100) verbunden ist, aufweist, und wobei die zweite Luftöffnung (13) auf einer von der Temperaturausgleichsplatte (42) abgewandten Seite des Wärmeableitungsblechs (43) angeordnet ist; und
eine Innenwand des Gehäuses (100) ferner einen dritten Wärmeableitungskanal (57) definiert, wobei ein Ende des dritten Wärmeableitungskanals (57) mit einem von der ersten Luftöffnung (413) entfernten Ende des ersten Wärmeableitungskanals (51) verbunden ist, und wobei das andere Ende des dritten Wärmeableitungskanals (57) und ein von der ersten Luftöffnung (413) entferntes Ende des zweiten Wärmeableitungskanals (52) beide mit der zweiten Luftöffnung (13) verbunden sind und an der Verbindungsstelle eine Bernoulli-Struktur bilden.

14. Hautbehandlungsvorrichtung nach Anspruch 13, wobei ein Auslass an einem Ende des dritten Wärmeableitungskanals (57), das nahe der zweiten Luftöffnung (13) liegt, in einer Innenwand an einem Ende des zweiten Wärmeableitungskanals (52), das nahe der zweiten Luftöffnung (13) liegt, definiert ist;
und/oder,
sich der zweite Wärmeableitungskanal (52) von der ersten Luftöffnung (413) des Lüfters (41) zu der zweiten Luftöffnung (13) des Gehäuses (100) erstreckt, das Wärmeableitungsblech (43) in dem zweiten Wärmeableitungskanal (52) angeordnet ist, und das Wärmeableitungsblech (43) sowohl an die erste Luftöffnung (413) als auch an die zweite Luftöffnung (13) angrenzt; und
der erste Wärmeableitungskanal (51) auf einer Seite der Temperaturausgleichsplatte (42), die von der zweiten Luftöffnung (13) abgewandt ist, angeordnet ist, sodass eine Gesamtlänge eines Strömungswegs der Luft in dem ersten Wärmeableitungskanal (51) und dem dritten Wärmeableitungskanal (57) größer ist als eine Gesamtlänge eines Strömungswegs der Luft in dem zweiten Wärmeableitungskanal (52);
und/oder
die Hautbehandlungsvorrichtung ferner eine Leiterplattenkomponente (600) aufweist, die Leiterplattenkomponente (600)
eine Leiterplatte (61), die in dem Gehäuse (100) angeordnet ist, wobei die Leiterplatte (61) und die Halterungskomponente (500) den dritten Wärmeableitungskanal (57) definieren; und eine leitfähige Halterung (62), die an der Leiterplatte (61) angebracht und in dem dritten Wärmeableitungskanal (57) angeordnet ist, aufweist;
wobei die Lichtemissionskomponente (200) eine Lichtröhre aufweist und ein Ende der Lichtröhre mit der leitfähigen Halterung (62) verbunden ist, sodass die Lichtröhre auf der Leiterplatte (61) abgestützt und elektrisch mit der Leiterplatte (61) verbunden ist.

15. Hautbehandlungsvorrichtung nach Anspruch 5, wobei eine Innenwand des ersten Wärmeableitungskanals (51) mit einer Montageöffnung (516) definiert ist, die Lichtemissionskomponente durch die Montageöffnung (516) in dem ersten Wärmeableitungskanal (51) angebracht ist, und die Lichtemissionskomponente (200) und eine Innenwand der Montageöffnung (516) eine Spaltluftöffnung (212) definieren; und wobei die Lichtemissionskomponente (200) ein Lichtreflexionselement (21) und eine Lichtquelle (22) aufweist, die Lichtquelle (22) in dem Lichtreflexionselement (21) angeordnet ist, eine seitliche Luftöffnung (213) an einer Umfangsseite des Lichtreflexionselements (21) definiert ist, die seitliche Luftöffnung (213) mit dem ersten Wärmeableitungskanal (51) verbunden ist, und ein Ende des Lichtreflexionselements (21) mit einer Endluftöffnung (214) definiert ist.

## Revendications

1. Appareil de traitement de la peau, comprenant :
un boîtier (100) muni d'une zone de sortie de lumière (11) ;
un composant émetteur de lumière (200) agencé dans le boîtier (100), le composant émetteur de lumière (200) étant configuré pour générer de la lumière irradiée sur une peau à traiter à partir de la zone de sortie de lumière (11) ;
un composant de compresse froide (300) disposé au niveau de la sortie de lumière (11) pour refroidir la peau ; et
un composant de dissipation de chaleur (400), comprenant :
un ventilateur (41) ;
une plaque d'égalisation de température (42) ; et
une feuille de dissipation de chaleur (43) montée sur la plaque d'égalisation de température (42) ;
dans lequel le ventilateur (41) est défini avec un premier évent d'aération (413),
**caractérisé en ce que** l'une des extrémités de la plaque d'égalisation de température (42) est reliée thermiquement au composant de compresse froide (300), et l'autre extrémité de la plaque d'égalisation de température (42) est située au niveau du premier évent d'aération (413) ; le ventilateur (41) entraîne une première partie de l'air à travers un côté de la plaque d'égalisation de température (42) opposé à la feuille de dissipation de chaleur (43) et à travers le composant émetteur de lumière (200) pour dissiper la chaleur dudit composant émetteur de lumière (200), et entraîne une seconde partie de l'air à travers la feuille de dissipation de chaleur (43) pour dissiper la chaleur du composant de compresse froide (300).

2. Appareil de traitement de la peau selon la revendication 1, comprenant en outre :
un élément de support (500) agencé dans le boîtier (100), l'élément de support (500) et la plaque d'égalisation de température (42) définissant un premier canal de dissipation de chaleur (51) pour laisser s'écouler la première partie de l'air ; dans lequel,
le composant émetteur de lumière (200) est au moins partiellement disposé dans le premier canal de dissipation de chaleur (51) ; et
l'élément de support (500) comprend :
une partie de séparation (55), la partie de séparation (55) séparant le composant émetteur de lumière (200) de la plaque d'égalisation de température (42).

3. Appareil de traitement de la peau selon la revendication 2, dans lequel,
la partie de séparation (55) est plus courte que la plaque d'égalisation de température (42) dans une direction du composant émetteur de lumière (200) faisant face au premier évent d'aération (413), et la partie de séparation (55) est supportée par la plaque d'égalisation de température (42) ; et/ou
une surface latérale de la plaque d'égalisation de température (42) faisant face au composant émetteur de lumière (200) comprend une zone abritée (422) et une zone exposée (423), la partie de séparation (55) est supportée sur la zone abritée (422), et une distance minimale entre la zone exposée (423) et le composant émetteur de lumière (200) est supérieure ou égale à 6 millimètres ; et/ou
une surface latérale de la plaque d'égalisation de température (42) faisant face au composant émetteur de lumière (200) comprend une zone abritée (422) et une zone exposée (423), la partie de séparation (55) est supportée sur la zone abritée (422), et la zone exposée (423) est une zone plate destinée à guider et à dissiper la chaleur ; et/ou
l'appareil de traitement de la peau est un dispositif d'épilation ou un dispositif de rajeunissement cutané.

4. Appareil de traitement de la peau selon la revendication 1, comprenant en outre :
un élément de support (500) agencé dans le boîtier (100), l'élément de support (500) et la plaque d'égalisation de température (42) définissant un premier canal de dissipation de chaleur (51) et un deuxième canal de dissipation de chaleur (52) ; dans lequel,
le composant émetteur de lumière (200) est au moins partiellement monté dans le premier canal de dissipation de chaleur (51) ; un côté de la plaque d'égalisation de température (42) et l'élément de support (500) définissent une section proche de l'évent d'aération du premier canal de dissipation de chaleur (51) ; cette section proche de l'évent d'aération du premier canal de dissipation de chaleur (51) communique avec le premier évent d'aération (413) ; et le ventilateur (41) entraîne la circulation d'une première partie de l'air à travers cette section proche de l'évent d'aération du premier canal de dissipation de chaleur (51) afin de dissiper la chaleur du composant émetteur de lumière (200) ; et
la feuille de dissipation de chaleur (43) est disposée dans le deuxième canal de dissipation de chaleur (52), l'autre côté de la plaque d'égalisation de température (42) et de l'élément de support (500) définissent une section d'évent d'aération proche du deuxième canal de dissipation de chaleur, la section d'évent d'aération proche du deuxième canal de dissipation de chaleur (52) communique avec le premier évent d'aération (413), et le ventilateur (41) entraîne la circulation de deuxième partie de l'air à travers la section d'évent d'aération proche du deuxième canal de dissipation de chaleur (52) pour dissiper la chaleur du composant de compresse froide (300).

5. Appareil de traitement de la peau selon la revendication 4, dans lequel le premier canal de dissipation de chaleur (51) comprend :
une section de montage (511) et une section de guidage d'écoulement (512) agencées le long d'une direction du composant émetteur de lumière (200) faisant face au premier évent d'aération (413) ; dans lequel,
le composant émetteur de lumière (200) est au moins partiellement monté dans la section de montage (511) ;
une extrémité de la section de guidage d'écoulement (512) est située au niveau du premier évent d'aération (413) pour être reliée au premier évent d'aération (413), et l'autre extrémité de la section de guidage d'écoulement (512) est reliée à la section de montage (511) ;
la section proche de l'évent d'aération du premier canal de dissipation thermique (51) est définie dans la section de guidage d'écoulement (512) ; et
une zone de section transversale d'au moins une région partielle de la section de guidage d'écoulement (512) augmente progressivement le long d'une direction du premier évent d'aération (413) faisant face au composant émetteur de lumière (200).

6. Appareil de traitement de la peau selon la revendication 5, dans lequel une paroi intérieure de la section de guidage d'écoulement (512) comprend :
une première paroi latérale intérieure (513) faisant face à la plaque d'égalisation de température (42) ; et
une deuxième paroi latérale intérieure (514) faisant face à la première paroi latérale intérieure (513) ;
dans lequel, dans la direction du premier évent d'aération (413) faisant face au composant émetteur de lumière (200), au moins l'une parmi la première paroi latérale intérieure (513) et la deuxième paroi latérale intérieure (514) est inclinée à l'opposé par rapport à l'autre, afin de permettre d'augmenter progressivement la distance entre la première paroi latérale intérieure (513) et la deuxième paroi latérale intérieure (514).

7. Appareil de traitement de la peau selon la revendication 6, dans lequel une première structure anti-turbulence (515) est prévue dans la section de guidage d'écoulement (512) pour limiter la formation de turbulence dans la section de guidage d'écoulement (512).

8. Appareil de traitement de la peau selon la revendication 7, dans lequel le composant émetteur de lumière (200) comprend :
un tube lumineux ; et
un élément réfléchissant la lumière (21), l'élément réfléchissant la lumière (21) comprenant une partie en forme d'arc (211) disposée autour du tube lumineux, et la partie en forme d'arc (211) étant au moins partiellement située sur un côté du tube lumineux proche de la section de guidage d'écoulement (512) ;
dans lequel une extrémité de la partie en forme d'arc (211) dans une direction périphérique est adjacente à la première paroi latérale intérieure (513), et l'autre extrémité de la partie en forme d'arc (211) dans une direction périphérique est adjacente à la deuxième paroi latérale intérieure (514) ; et
la première paroi latérale intérieure (513) ou la deuxième paroi latérale intérieure (514) est munie de la première structure anti-turbulence (515), ou chacune de la première paroi latérale intérieure (513) et de la deuxième paroi latérale intérieure (514) est munie de la première structure anti-turbulence (515) ;
et/ou,
la première structure anti-turbulence (515) comprend une plaque de guidage d'écoulement, dans lequel,
la plaque de guidage d'écoulement s'étend dans la direction du premier évent d'aération (413) faisant face au composant émetteur de lumière (200) ; et/ou
la plaque de guidage d'écoulement est l'une d'une pluralité de plaques de guidage d'écoulement, et la pluralité de plaques de guidage d'écoulement sont espacées le long d'une direction de la longueur du composant émetteur de lumière (200) ; et/ou
la plaque de guidage d'écoulement comprend une première surface inclinée (5151) et une deuxième surface inclinée (5152) se faisant face dans le sens de l'épaisseur de la plaque de guidage d'écoulement ; la première surface inclinée (5151) et la deuxième surface inclinée (5152) sont inclinées l'une par rapport à l'autre dans le sens du premier évent d'aération (413) faisant face au composant émetteur de lumière (200), afin de permettre à l'épaisseur d'une extrémité de la plaque de guidage d'écoulement proche du composant émetteur de lumière (200) d'être supérieure à l'épaisseur d'une extrémité de la plaque de guidage d'écoulement éloignée du composant émetteur de lumière (200) ; et/ou
la paroi intérieure de la section de guidage d'écoulement (512) comprend une première surface de guidage d'écoulement reliée à la plaque de guidage d'écoulement, ladite plaque de guidage d'écoulement comprend en outre une troisième surface inclinée (5153), la troisième surface inclinée (5153) est située sur un côté de la plaque de guidage d'écoulement opposé à la première surface de guidage d'écoulement ; la troisième surface inclinée (5153) est inclinée par rapport à la première surface de guidage d'écoulement, dans la direction du premier évent d'aération (413) faisant face au composant émetteur de lumière (200), afin que la hauteur de la plaque de guidage d'écoulement dépassant de la première surface de guidage d'écoulement à une extrémité de la plaque de guidage d'écoulement proche du composant émetteur de lumière (200) soit supérieure à la hauteur de la plaque de guidage d'écoulement dépassant de la première surface de guidage d'écoulement à une extrémité de la plaque de guidage d'écoulement opposée au composant émetteur de lumière (200).

9. Appareil de traitement de la peau selon la revendication 5, dans lequel une extrémité de la plaque d'égalisation de température (42) proche du premier évent d'aération (413) est déviée vers un côté proche du composant émetteur de lumière (200), pour former une section de déviation (421) ;
dans lequel, la section de déviation (421) s'étend jusqu'au premier évent d'aération (413), un côté de la section de déviation (421) proche du composant émetteur de lumière (200) et l'élément de support (500) définissent au moins une partie de la section de guidage d'écoulement (512), et un côté de la section de déviation (421) faisant face à l'opposé du composant émetteur de lumière (200) et l'élément de support (500) définissent la section proche de l'évent d'aération du deuxième canal de dissipation de chaleur (52).

10. Appareil de traitement de la peau selon la revendication 9, dans lequel la feuille de dissipation de chaleur (43) comprend :
une section de dissipation de chaleur proximale (431) montée sur la section de déviation (421) ; et
une section de dissipation de chaleur distale (432) reliée à une extrémité de la section de dissipation de chaleur proximale (431) proche de la zone de sortie de lumière (11) ;
dans lequel la largeur de la section de dissipation de chaleur proximale (431) est supérieure à la largeur de la section de dissipation de chaleur distale (432), et la largeur de la feuille de dissipation de chaleur (43) est une distance d'un côté de la feuille de dissipation de chaleur (43) proche de la plaque d'égalisation de température (42) à un côté de la feuille de dissipation de chaleur (43) éloigné de la plaque d'égalisation de température (42).

11. Appareil de traitement de la peau selon la revendication 10, dans lequel la feuille de dissipation de chaleur (43) comprend :
un bord latéral de connexion (433) monté sur la plaque d'égalisation de température ; et
un bord latéral libre (434) orienté à l'opposé de la plaque d'égalisation de température (42) ;
dans lequel, dans la direction du composant émetteur de lumière (200) faisant face au premier évent d'aération (413), le bord latéral de connexion (433) et le bord latéral libre (434) de la section de dissipation de chaleur proximale (431) sont inclinés l'un par rapport à l'autre, pour permettre d'augmenter progressivement la largeur de la section de dissipation de chaleur proximale (431) ; et/ou,
le boîtier (100) comprend :
une section de sortie de lumière s'étendant d'une jonction de la section de dissipation de chaleur proximale (431) et de la section de dissipation de chaleur distale (432) à la zone de sortie de lumière (11) ;
dans lequel la section transversale de la section de sortie de lumière diminue progressivement le long d'une direction du composant émetteur de lumière (200) faisant face à la zone de sortie de lumière (11).

12. Appareil de traitement de la peau selon la revendication 10, dans lequel la feuille de dissipation de chaleur (43) est l'une d'une pluralité de feuilles de dissipation de chaleur (43), chaque feuille de dissipation de chaleur (43) comprend le bord latéral de connexion (433) monté sur la plaque d'égalisation de température (42) et le bord latéral libre (434) faisant face à l'opposé de la plaque d'égalisation de température (42), les bords latéraux libres (434) de la pluralité de feuilles de dissipation de chaleur (43) définissent une surface latérale libre, et la surface latérale libre comprend une zone de blocage d'air (435) près du premier évent d'aération (413) et une zone de circulation (436) près du composant de compresse froide (300) ;
dans lequel le composant de dissipation de chaleur (400) comprend en outre une partie de blocage (44), la partie de blocage (44) bloque la zone de blocage d'air (435), et l'air entre les feuilles de dissipation de chaleur adjacentes (43) s'écoule vers l'extérieur ou vers l'intérieur de la zone de circulation (436) près du composant de compresse froide (300).

13. Appareil de traitement de la peau selon la revendication 5, dans lequel,
le boîtier (100) est en outre défini par un deuxième évent d'aération (13) communiquant avec l'extérieur du boîtier (100), et ce deuxième évent d'aération (13) est situé sur un côté de la plaque de dissipation de chaleur (43) faisant face à la plaque d'égalisation de température (42) ; et
une paroi intérieure du boîtier (100) définit en outre un troisième canal de dissipation de chaleur (57), une extrémité du troisième canal de dissipation de chaleur (57) est en communication avec une extrémité du premier canal de dissipation de chaleur (51) éloignée du premier évent d'aération (413), et l'autre extrémité du troisième canal de dissipation de chaleur (57) et une extrémité du deuxième canal de dissipation de chaleur (52) éloignée du premier évent d'aération (413) sont toutes deux en communication avec le deuxième évent d'aération (13) et forment une structure de Bernoulli à la jonction.

14. Appareil de traitement de la peau selon la revendication 13, dans lequel une sortie à une extrémité du troisième canal de dissipation de chaleur (57) près du deuxième évent d'aération (13) est définie dans une paroi intérieure à une extrémité du deuxième canal de dissipation de chaleur (52) près du deuxième évent d'aération (13) ;
et/ou,
le deuxième canal de dissipation de chaleur (52) s'étend du premier évent d'aération (413) du ventilateur (41) au deuxième évent d'aération (13) du boîtier (100), la feuille de dissipation de chaleur (43) est disposée dans le deuxième canal de dissipation de chaleur (52), et la feuille de dissipation de chaleur (43) est adjacente à la fois au premier évent d'aération (413) et au deuxième évent d'aération (13) ; et
le premier canal de dissipation de chaleur (51) est situé sur un côté de la plaque d'égalisation de température (42) faisant face au deuxième évent d'aération (13), pour permettre à la longueur totale d'un trajet d'écoulement d'air dans le premier canal de dissipation de chaleur (51) et le troisième canal de dissipation de chaleur (57) d'être supérieure à la longueur totale d'un trajet d'écoulement d'air dans le deuxième canal de dissipation de chaleur (52) ; et/ou,
l'appareil de traitement de la peau comprend en outre un composant de circuit imprimé (600), ce composant de circuit imprimé (600) comprenant :
une carte de circuit imprimé (61) disposée dans le boîtier (100), la carte de circuit imprimé (61) et l'élément de support (500) définissant le troisième canal de dissipation de chaleur (57) ; et
un support conducteur (62) monté sur la carte de circuit imprimé (61) et situé dans le troisième canal de dissipation de chaleur (57) ;
dans lequel le composant émetteur de lumière (200) comprend un tube lumineux, et une extrémité du tube lumineux est connectée au support conducteur (62), pour permettre au tube lumineux d'être supporté sur la carte de circuit imprimé (61) et connecté électriquement à la carte de circuit imprimé (61).

15. Appareil de traitement de la peau selon la revendication 5, dans lequel une paroi intérieure du premier canal de dissipation de chaleur (51) est définie par un orifice de montage (516), le composant émetteur de lumière est monté dans le premier canal de dissipation de chaleur (51) à travers l'orifice de montage (516), et le composant émetteur de lumière (200) et une paroi intérieure de l'orifice de montage (516) définissent un évent d'espace d'aération (212) ; et
le composant émetteur de lumière (200) comprend un élément réfléchissant la lumière (21) et une source de lumière (22), la source de lumière (22) est agencée dans l'élément réfléchissant la lumière (21), un évent d'aération latéral (213) est défini sur un côté périphérique de l'élément réfléchissant la lumière (21), l'évent d'aération latéral (213) est en communication avec le premier canal de dissipation de chaleur (51), et une extrémité de l'élément réfléchissant la lumière (21) est définie avec un évent d'aération d'extrémité (214).
